# EUROPEAN PATENT APPLICATION

(11) **EP 3 563 817 A1**
(43) Date of publication of application: **06.11.2019**
(21) Application number: 16925233.5
(22) Date of filing: 27.12.2016
(51) Int. Cl.: A61F 13/15, A61F 13/49

(54) **PRODUCTION METHOD AND PRODUCTION DEVICE FOR SHEET-LIKE MEMBER FOR ABSORBENT ARTICLE**

(71) Applicant: Unicharm Corporation, Shikokuchuo-shi, Ehime 799-0111 (JP)
(72) Inventor: NINOMIYA, Akihide, Kanonji-shi Kagawa 769-1602 (JP); TOMIOKA, Masaharu, Kanonji-shi Kagawa 769-1602 (JP); NOMA, Shinji, Kanonji-shi Kagawa 769-1602 (JP)
(74) Representative: Staeger & Sperling Partnerschaftsgesellschaft mbB
(86) International application number: PCT/JP2016/088928
(87) International publication number: WO 2018/122970

(57) **Abstract**

There is provided a method for manufacturing a sheet-like member having an elastic member (35) attached to the sheet-like member. The sheet-like member is manufactured by inserting an elastic-member continuous body (35a) between a pair of mutually-opposing facing surfaces of a sheet-like member continuous body being continuous in the direction of transport, and then cutting the sheet-like member continuous body. The method includes: a fixing step of fixing to the facing surface with an adhesive (BD) the elastic-member continuous body being stretched; a forming step of forming joining portions (j) that join the pair of facing surfaces to each other; and a cutting step of producing the sheet-like member and the elastic member by cutting the sheet-like member continuous body and the elastic-member continuous body. In the fixing step, a portion (35ae) of the elastic-member continuous body that is to be an end portion (35e) of the elastic member is fixed to the at least one facing surface with use of the adhesive. In the forming step, the joining portions are formed on two sides of the elastic-member continuous body in a CD direction. In the cutting step, the elastic member that attempts to expand in the CD direction due to being cut is attached to the sheet-like member by being sandwiched and pressed in the CD direction between the joining portions on the two sides.

## Description

### [Technical Field]

The present invention relates to a method and a device for manufacturing a sheet-like member for an absorbent article such as a disposable diaper.

### [Background Art]

A disposable diaper is a conventional example of an absorbent article that absorbs excrement such as urine. Such a diaper is manufactured using sheet-like members that are given stretchability by elastic members being attached thereto with use of an adhesive such as hot-melt adhesive.

However, when the elastic members are attached using an adhesive, the elasticity (i.e., stretchability) of the elastic members is impaired by the hardening of the adhesive that is located on the outer circumferential surfaces of the elastic members, thus leading to the risk of impairing the softness of the sheet-like member. For this reason, in recent years, consideration has been given to the attachment of elastic members to a sheet-like member without using an adhesive, and the following is one example of such a method disclosed in PTL 1.

### [Citation List]

### [Patent Literature]

[PTL 1] Japanese Patent No. 3212615

### [Summary of Invention]

### [Technical Problem]

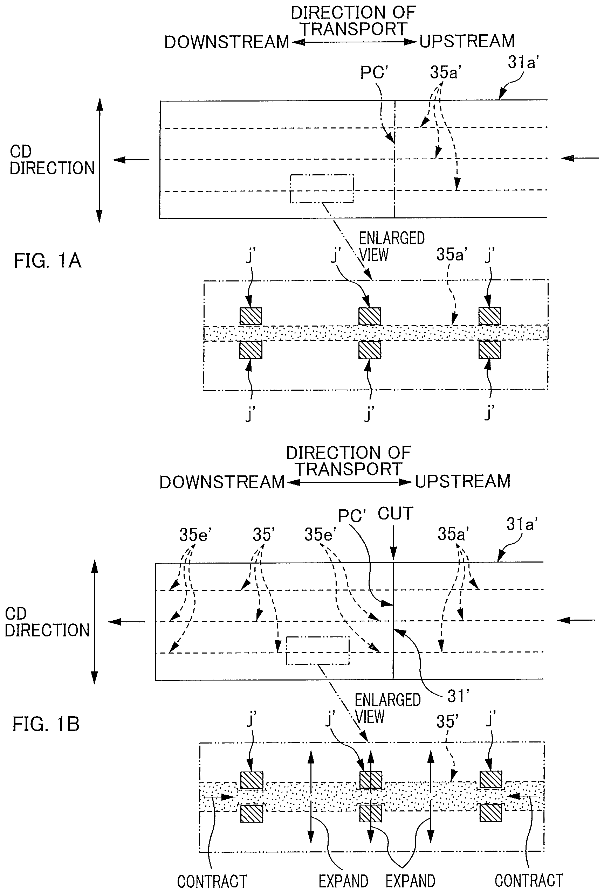

First, as shown in the schematic plan view of FIG. 1A, elastic-member continuous bodies 35a', which are continuous in a direction of transport, are stretched in the direction of transport and inserted in that state between a pair of mutually-opposing facing surfaces of sheet-like-member continuous bodies 31a' that are continuous in the direction of transport.

Next, a plurality of joining portions j' for joining the pair of facing surfaces are formed with gaps therebetween in the direction of transport, and at this time, the joining portions j' are formed at positions on two sides of the elastic-member continuous bodies 35a' with respect to a CD direction that intersects the direction of transport.

Subsequently, in the continuous bodies 31a', the sheet-like-member continuous bodies 31a' are cut at cutting target positions PC' in the direction of transport, thus forming single-cut sheet-like members 31' having the elastic members 35' attached thereto as shown in FIG. 1B. Due to this cutting, the cut elastic members 35' contract in the direction of transport and attempt to expand in the CD direction, but here, the expansion of the elastic members 35' in the CD direction is restricted by the pairs of joining portions j' located on the two sides in the CD direction, and therefore the elastic members 35' are substantially sandwiched and pressed in the CD direction between the joining portions j'. As a result, the elastic members 35' become attached to the sheet-like member 31'.

However, these elastic members 35' are attached to the sheet-like members 31' basically due to simply being sandwiched and pressed between pairs of the joining portions j' as described above. The strength of this attachment through pressing is smaller than the strength of attachment through an adhesive. For this reason, end portions 35e' in the direction of transport of the elastic members 35' in FIG. 1B can easily become separated from the pair of joining portions j', and as a result, it has been difficult to give the sheet-like members 31' stable stretchability up to the end portions 35e'.

The present invention was achieved in light of conventional problems such as that described above, and an aspect of the present invention is to suppress a case where the end portions in the direction of transport of the elastic members become separated from the sheet-like members.

### [Solution to Problem]

A main aspect of the present invention for achieving the above-described aspect is a method for manufacturing a sheet-like member for an absorbent article,
the sheet-like member having an elastic member attached to the sheet-like member,
the sheet-like member being manufactured by inserting an elastic-member continuous body between a pair of mutually-opposing facing surfaces of a sheet-like member continuous body, and then cutting the sheet-like member continuous body at a cutting target position in a direction of transport of the sheet-like member continuous body,
the elastic-member continuous body being continuous in the direction of transport,
the sheet-like member continuous body being continuous in the direction of transport,
the method including:
a fixing step of fixing the elastic-member continuous body to at least one facing surface out of the pair of facing surfaces with use of an adhesive,
   the elastic-member continuous body being stretched in the direction of transport,
   the fixing step including fixing a portion of the elastic-member continuous body to the at least one facing surface with use of the adhesive,
      the portion being a portion that is to be an end portion of the elastic member in the direction of transport when cutting the elastic-member continuous body at the cutting target position;
a joining-portion forming step of forming a plurality of joining portions spacing in the direction of transport,
   the joining portions joining the pair of facing surfaces to each other,
   the joining-portion forming step including forming the plurality of joining portions at positions on two sides of the elastic-member continuous body in a CD direction that intersects the direction of transport; and
a cutting step of producing the sheet-like member and the elastic member by cutting the sheet-like member continuous body and the elastic-member continuous body at the cutting target position after the fixing step and the joining-portion forming step,
   the cutting step including attaching the elastic member to the sheet-like member by being sandwiched and pressed in the CD direction between the joining portions on the two sides,
   the elastic member contracting in the direction of transport and attempting to expand in the CD direction due to being cut at the cutting target position.

Features of the present invention other than the above will become clear by reading the description of the present specification with reference to the accompanying drawings.

### [Advantageous Effects of Invention]

According to the present invention, it is possible to suppress a case where the end portions of the elastic members in the direction of transport become separated from the sheet-like members.

### [Brief Description of the Drawings]

FIGS. 1A and 1B are illustrative diagrams of a method for attaching elastic members 35' to sheet-like members 31' without using an adhesive.
FIG. 2 is a schematic perspective view of a three piece-type of diaper 1 as one example of an absorbent article.
FIG. 3 is a schematic plan view of the diaper 1 in an unfolded state as viewed from a wearer's skin side.
FIG. 4 includes a cross-sectional view along IVa-Iva and a cross-sectional view along IVb-IVb in FIG. 3.
FIG. 5 is a schematic plan view of a front band member 31 in an unfolded state as viewed from a non-skin side.
FIGS. 6A and 6B are illustrative views of an elastic string 35 (45) attachment function exhibited by welded portions j.
FIG. 7 is a schematic plan view showing a partial perspective view of how the diaper 1 is manufactured in a manufacturing line.
FIGS. 8A, 8B, and 8C are respectively schematic enlarged views of portions A, B, and C in FIG. 7.
FIG. 9A is an illustrative view of an example of a variation of application regions ABD of an adhesive BD for fixing end portions 35e (45e) of elastic strings 35 (45).
FIG. 9B is an illustrative diagram of another example of a variation.
FIG. 9C is an illustrative diagram of another example of a variation.
FIG. 9D is an illustrative diagram of another example of a variation.
FIG. 10A is an illustrative diagram of another example of a variation.
FIG. 10B is an illustrative diagram of another example of a variation.
FIG. 10C is an illustrative diagram of another example of a variation.
FIG. 11 is an illustrative diagram of an example in which a welded portion SSk of a downstream side-seal section SSd is provided so as to be overlapped with an application region ABD.
FIG. 12 is an illustrative diagram of an example in which a welded portion pair jP is added so as to be overlapped with an application region ABD.

### [Description of Embodiments]

At least the following matters will become clear with the description of this specification and the attached drawings. A method for manufacturing a sheet-like member for an absorbent article,
the sheet-like member having an elastic member attached to the sheet-like member,
the sheet-like member being manufactured by inserting an elastic-member continuous body between a pair of mutually-opposing facing surfaces of a sheet-like member continuous body, and then cutting the sheet-like member continuous body at a cutting target position in a direction of transport of the sheet-like member continuous body,
the elastic-member continuous body being continuous in the direction of transport,
the sheet-like member continuous body being continuous in the direction of transport,
the method including:
a fixing step of fixing the elastic-member continuous body to at least one facing surface out of the pair of facing surfaces with use of an adhesive,
   the elastic-member continuous body being stretched in the direction of transport,
   the fixing step including fixing a portion of the elastic-member continuous body to the at least one facing surface with use of the adhesive,
      the portion being a portion that is to be an end portion of the elastic member in the direction of transport when cutting the elastic-member continuous body at the cutting target position;
a joining-portion forming step of forming a plurality of joining portions spacing in the direction of transport,
   the joining portions joining the pair of facing surfaces to each other,
   the joining-portion forming step including forming the plurality of joining portions at positions on two sides of the elastic-member continuous body in a CD direction that intersects the direction of transport; and
a cutting step of producing the sheet-like member and the elastic member by cutting the sheet-like member continuous body and the elastic-member continuous body at the cutting target position after the fixing step and the joining-portion forming step,
   the cutting step including attaching the elastic member to the sheet-like member by being sandwiched and pressed in the CD direction between the joining portions on the two sides,
   the elastic member contracting in the direction of transport and attempting to expand in the CD direction due to being cut at the cutting target position.

According to this method for manufacturing a sheet-like member for an absorbent article, the end portion of the elastic member in the direction of transport is firmly fixed to the sheet-like member with use of the adhesive. Accordingly, it is possible to suppress the case where the end portion becomes separated from the sheet-like member.

In such a method for manufacturing a sheet-like member for an absorbent article, it is desirable
that the method further comprises a welding step of forming a welded portion on two sides of the cutting target position in the direction of transport,
the welded portion being a portion that joins the pair of facing surfaces to each other,
that the adhesive is applied in a predetermined application region of at least either of the elastic-member continuous body and one of the pair of facing surfaces, and
that concerning a downstream welded portion being one of the welded portions on the two sides and being located on a downstream side in the direction of transport,
a downstream end of the application region in the direction of transport is located downstream in the direction of transport with respect to a downstream end of the downstream welded portion in the direction of transport.

According to this method for manufacturing a sheet-like member for an absorbent article, the downstream end of the application region of the adhesive is located downstream with respect to the downstream end of the downstream welded portion. Accordingly, a larger size in the direction of transport can be ensured for the application region compared to the case where the downstream end of the application region is located upstream with respect to the downstream welded portion. Accordingly, the end portion of the elastic member in the direction of transport can be fixed more firmly by the adhesive, thus making it possible to reliably suppress the case where the end portion becomes separated from the sheet-like member.

In such a method for manufacturing a sheet-like member for an absorbent article, it is desirable that an upstream end of the application region in the direction of transport is located downstream in the direction of transport with respect to the downstream end of the downstream welded portion.

According to this method for manufacturing a sheet-like member for an absorbent article, the upstream end of the application region of the adhesive is located downstream with respect to the downstream end of the downstream welded portion. Accordingly, a smaller size in the direction of transport can be set for the application region compared to the case where the upstream end of the application region is located upstream with respect to the downstream end of the downstream welded portion. Accordingly, it is possible to prevent a problem that can occur if the application region is too large, such as the stretchability of the elastic member or the softness of the sheet-like member being impaired by hardening of the adhesive .

In such a method for manufacturing a sheet-like member for an absorbent article, it is desirable that
an upstream end of the application region in the direction of transport is located in the direction of transport between the downstream end of the downstream welded portion and an upstream end of the downstream welded portion in the direction of transport.

According to this method for manufacturing a sheet-like member for an absorbent article, the upstream end of the adhesive application region is located between the downstream end and the upstream end of the downstream welded portion. Accordingly, a larger size in the direction of transport can be ensured for the application region compared to the case where the upstream end of the application region is located downstream with respect to the downstream end of the downstream welded portion. Accordingly, the end portion of the elastic member in the direction of transport can be fixed more firmly by the adhesive, thus making it possible to reliably suppress the case where the end portion becomes separated from the sheet-like member.

In such a method for manufacturing a sheet-like member for an absorbent article, it is desirable that
an upstream end of the application region in the direction of transport is located in the direction of transport between the cutting target position and an upstream end of the downstream welded portion in the direction of transport.

According to this method for manufacturing a sheet-like member for an absorbent article, the upstream end of the application region of the adhesive is located between the upstream end of the downstream welded portion and the cutting target position. Accordingly, a larger size in the direction of transport can be ensured for the application region compared to the case where the upstream end of the application region is located downstream with respect to the upstream end of the downstream welded portion. Accordingly, the end portion of the elastic member in the direction of transport can be fixed more firmly by the adhesive, thus making it possible to reliably suppress the case where the end portion becomes separated from the sheet-like member.

In such a method for manufacturing a sheet-like member for an absorbent article, it is desirable that
an upstream end of the application region in the direction of transport is located upstream in the direction of transport with respect to the cutting target position.

According to this method for manufacturing a sheet-like member for an absorbent article, the upstream end of the application region of the adhesive is located upstream with respect to the cutting target position. Accordingly, in the sheet-like member, the size of the application region on the upstream side can be maximized, thus making it possible for the end portion of the elastic member in the direction of transport to be more firmly fixed by the adhesive .

In such a method for manufacturing a sheet-like member for an absorbent article, it is desirable
that the method further comprises a welding step of forming a welded portion on two sides of the cutting target position in the direction of transport,
the welded portion being a portion that joins the pair of facing surfaces to each other,
that the adhesive is applied in a predetermined application region of at least either of the elastic-member continuous body and one of the pair of facing surfaces, and
that concerning a downstream welded portion being out of the welded portions on the two sides and being located on a downstream side in the direction of transport,
a downstream end of the application region in the direction of transport is located on upstream in the direction of transport with respect to a downstream end of the downstream welded portion in the direction of transport.

According to this method for manufacturing a sheet-like member for an absorbent article, the downstream end of the application region of the adhesive is located upstream with respect to the downstream end of the downstream welded portion. Accordingly, the application region can be overall set closer to the cutting target position in the direction of transport compared to the case where the downstream end of the application region is located downstream with respect to the downstream end of the downstream welded portion. Accordingly, the portion in which the stretchability of the elastic member is impaired by hardening of the adhesive can be positioned closer to the cutting target position, thus making it possible to prevent the portion in which the stretchability of the elastic member is impaired by hardening of the adhesive from becoming noticeable.

In such a method for manufacturing a sheet-like member for an absorbent article, it is desirable that
an upstream end of the application region in the direction of transport is located downstream in the direction of transport with respect to an upstream end of the downstream welded portion in the direction of transport.

According to this method for manufacturing a sheet-like member for an absorbent article, the downstream end of the application region of the adhesive is located downstream with respect to the upstream end of the downstream welded portion. Accordingly, a smaller size in the direction of transport can be set for the application region compared to the case where the upstream end of the application region is located upstream with respect to the upstream end of the downstream welded portion. Accordingly, it is possible to prevent a problem that can occur if the application region is too large, such as the stretchability of the elastic member or the softness of the sheet-like member being impaired by hardening of the adhesive.

In such a method for manufacturing a sheet-like member for an absorbent article, it is desirable that
an upstream end of the application region in the direction of transport is located in the direction of transport between the cutting target position and an upstream end of the downstream welded portion in the direction of transport.

According to this method for manufacturing a sheet-like member for an absorbent article, the upstream end of the application region of the adhesive is located between the cutting target position and the upstream end of the downstream welded portion. Accordingly, a larger size in the direction of transport can be ensured for the application region compared to the case where the upstream end of the application region is located downstream with respect to the upstream end of the downstream welded portion. Accordingly, the end portion of the elastic member in the direction of transport can be fixed more firmly by the adhesive, thus making it possible to reliably suppress the case where the end portion becomes separated from the sheet-like member.

In such a method for manufacturing a sheet-like member for an absorbent article, it is desirable that
an upstream end of the application region in the direction of transport is located upstream in the direction of transport with respect to the cutting target position.

According to this method for manufacturing a sheet-like member for an absorbent article, the upstream end of the application region of the adhesive is located upstream with respect to the cutting target position. Accordingly, in the sheet-like member, the size of the application region on the upstream side can be maximized, thus making it possible for the end portion of the elastic member in the direction of transport to be more firmly fixed by the adhesive .

In such a method for manufacturing a sheet-like member for an absorbent article, it is desirable that
the welded portions are provided so as to be overlapped with the application region when viewing the sheet-like member continuous body in a thickness direction that is orthogonal to both the direction of transport and the CD direction.

According to this method for manufacturing a sheet-like member for an absorbent article, the welded portion is provided so as to be overlapped with the application region of the adhesive. Accordingly, the welded portion can also contribute to fixing the end portion of the elastic member in the direction of transport to the sheet-like member with use of the adhesive, thus improving the fixed strength of the end portion of the elastic member.

In such a method for manufacturing a sheet-like member for an absorbent article, it is desirable that
at least one joining portion out of the joining portions is provided so as to be overlapped with the application region when viewing the sheet-like member continuous body in a thickness direction that is orthogonal to both the direction of transport and the CD direction.

According to this method for manufacturing a sheet-like member for an absorbent article, at least one of the joining portions is provided so as to be overlapped with the application region of the adhesive. Accordingly, the pressing by the joining portions can also contribute to fixing the end portion in the direction of transport of the elastic member to the sheet-like member with use of the adhesive, thus improving the fixed strength of the end portion of the elastic member.

In such a method for manufacturing a sheet-like member for an absorbent article, it is desirable
that a plurality of the elastic-member continuous bodies are arranged side-by-side spacing between elastic-member continuous bodies that are adjacent in the CD direction,
that in the fixing step, for each of the plurality of elastic-member continuous bodies,
   the portion of the elastic-member continuous body that is to be the end portion of the elastic member in the direction of transport when cutting the elastic-member continuous body at the cutting target position is fixed to the at least one facing surface with use of the adhesive,
that in the joining-portion forming step,
   the joining portions respectively corresponds to the plurality of elastic-member continuous bodies, and
among the plurality of elastic-member continuous bodies, when one is defined as a first elastic-member continuous body, and another one is defined as a second elastic-member continuous body,
   a tension (N) of the first elastic-member continuous body in the state of being stretched is higher than a tension (N) of the second elastic-member continuous body in the state of being stretched, and
   a length in the direction of transport of the application region of the first elastic-member continuous body is larger than a length in the direction of transport of the application region of the second elastic-member continuous body.

According to this method for manufacturing a sheet-like member for an absorbent article, the length in the direction of transport of the application region of the second elastic member, which has a higher tension than the first elastic member, is set longer than the length in the direction of transport of the application region of the first elastic member. Accordingly, concerning the second elastic member having a higher tension, its end portion can also be reliably fixed to the sheet-like member.

A device for manufacturing a sheet-like member for an absorbent article,
the sheet-like member having an elastic member attached to the sheet-like member,
the sheet-like member being manufactured by inserting an elastic-member continuous body between a pair of mutually-opposing facing surfaces of a sheet-like member continuous body, and then cutting the sheet-like member continuous body at a cutting target position in a direction of transport of the sheet-like member continuous body,
the elastic-member continuous body being continuous in the direction of transport,
the sheet-like member continuous body being continuous in the direction of transport
the device including:
an adhesive application device that discharges an adhesive in order to fix the elastic-member continuous body to at least one facing surface out of the pair of facing surfaces with use of the adhesive,
   the elastic-member continuous body being stretched in the direction of transport,
   the adhesive application device discharging the adhesive so as to fix a portion of the elastic-member continuous body to the at least one facing surface with use of the adhesive,
      the portion being a portion that is to be an end portion of the elastic member in the direction of transport when cutting the elastic-member continuous body at the cutting target position;
a joining-portion forming device that forms a plurality of joining portions spacing in the direction of transport, the joining portions joining the pair of facing surfaces to each other,
   the joining-portion forming device forming the plurality of joining portions at positions on two sides of the elastic-member continuous body in a CD direction that intersects the direction of transport; and
a cutting device that produces the sheet-like member by cutting the sheet-like member continuous body at the cutting target position when the sheet-like member continuous body is located at a position downstream in the direction of transport with respect to the adhesive application device and the joining-portion forming device,
the elastic member being attached to the sheet-like member by being sandwiched and pressed in the CD direction between the joining portions on the two sides,
when the attaching, the elastic member being in a state of contracting in the direction of transport and attempting to expand in the CD direction due to being cut at the cutting target position by the cutting device.

According to this device for manufacturing a sheet-like member for an absorbent article, it is possible to achieve actions and effects similar to those in the case of the manufacturing method described above.

### Embodiment

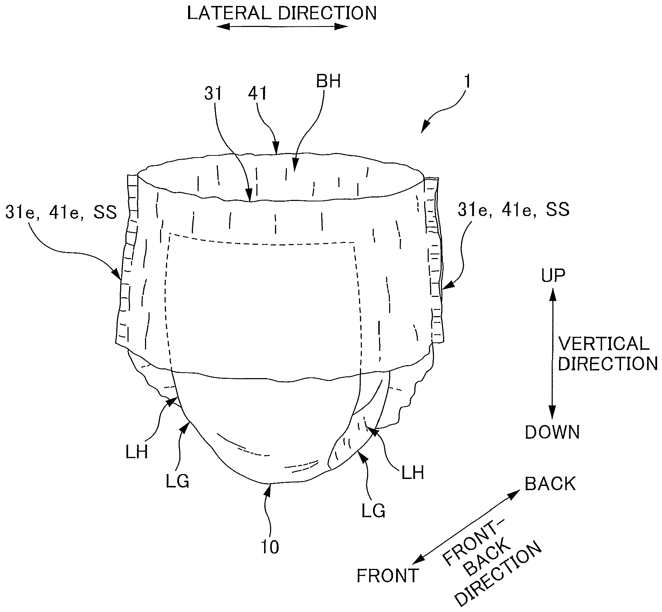

A method and a device for manufacturing a sheet-like member for an absorbent article according to an embodiment of the present invention are used in, for example, a manufacturing line for a disposable diaper 1 that is one example of an absorbent article. FIG. 2 is a schematic perspective view of a three piece-type of diaper 1 as one example of the diaper 1.

In the underpants-shaped state before being worn as shown in FIG. 2, the diaper 1 has a vertical direction, a lateral direction that is orthogonal to the vertical direction, and a "front-back direction" that is orthogonal to the vertical direction and the lateral direction. The vertical direction often conforms to the up-down direction when the diaper 1 is worn. For this reason, hereinafter, the vertical direction will also be called the up-down direction.

Note that with respect to the up-down direction, the upper side corresponds to the waist side of the wearer, and the lower side corresponds to the crotch side of the wearer. Also, with respect to the front-back direction, the front side corresponds to the stomach side of the wearer, and the back side corresponds to the back side of the wearer. Furthermore, with respect to the lateral direction, one side corresponds to the left side of the wearer, and the other side corresponds to the right side of the wearer.

In the underpants-shaped state shown in FIG. 2, the diaper 1 includes: a front band member 31 that extends in the lateral direction; a back band member 41 that extends along the lateral direction, is located on the back side of the front band member 31, and is for forming a waist opening BH on the upper side in the vertical direction along with the front band member 31; and an absorbent main body 10 that is a crotch portion provided between the front band member 31 and the back band member 41. The absorbent main body 10 is arranged at a position protruding farther downward in the vertical direction than the front band member 31 and the back band member 41 do.

Also, lateral end portions 31e of the front band member 31 and corresponding lateral end portions 41e of the back band member 41 are joined in side-seal sections SS that are welded portions. Accordingly, the front band member 31 and the back band member 41 respectively form leg openings LH on two lateral sides on the lower side along with the absorbent main body 10.

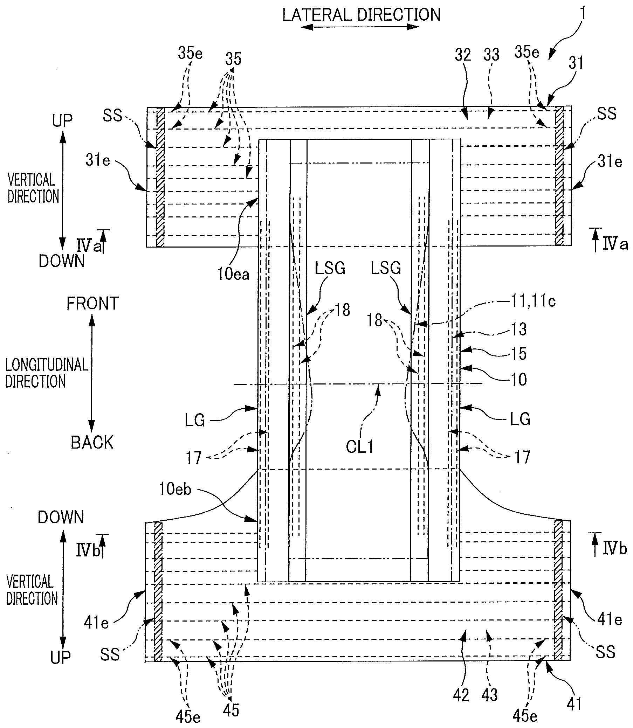

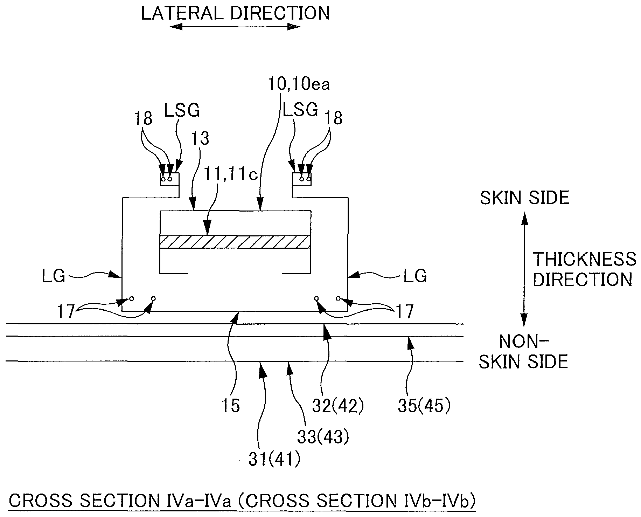

FIG. 3 is a schematic plan view of the diaper 1 in an unfolded state as viewed from the wearer's skin side. Also, FIG. 4 includes a cross-sectional view along IVa-IVa and a cross-sectional view along IVb-IVb in FIG. 3.

Here, the unfolded state is a state in which the aforementioned side-seal sections SS on the two lateral sides of the diaper 1 in the underpants-shaped state shown in FIG. 2 are detached such that the front band member 31 and the back band member 41 separate from each other and the diaper 1 is opened in the vertical direction, and thus the diaper 1 is unfolded in a plan view.

Also, in this unfolded state, the diaper 1 is shown in a virtual state in which there is no stretchability in the members that constitute the diaper 1. For example, although the diaper 1 is provided with elastic members 17, 18, 35, and 45 for the purpose of giving stretchability to the diaper 1 in this example, in the aforementioned unfolded state, the diaper 1 is shown in a virtual state in which the elastic members 17, 18, 35, and 45 have no stretchability (contractive force) whatsoever.

In the unfolded state, the diaper 1 has a longitudinal direction, a lateral direction, and a thickness direction (direction passing through the paper plane in FIG. 3) as three directions that are orthogonal to each other. Note that the longitudinal direction conforms the previously described vertical direction. Also, with respect to the longitudinal direction, one side corresponds to the stomach side, and the other side corresponds to the back side. Also, the outer side in the longitudinal direction corresponds to the upper side in the vertical direction, and the inner side in the longitudinal direction corresponds to the lower side in the vertical direction. Given that the longitudinal direction and the vertical direction are directions that resemble each other in this way, for the sake of convenience hereinafter, the vertical direction will sometimes be used in place of the longitudinal direction in the unfolded state as well. Furthermore, the lateral direction is synonymous with the lateral direction in the previously described underpants-shaped state. Moreover, with respect to the thickness direction, one side corresponds to the skin side that comes into contact with the wearer's body, and the other side corresponds to the opposite non-skin side. Note that the thickness direction conforms to the previously described front-back direction.

In the unfolded state shown in FIG. 3, the front band member 31 is arranged extending in the lateral direction, and the back band member 41 is arranged extending in the lateral direction at a position that is separated from the front band member 31 by a predetermined gap in the longitudinal direction. The absorbent main body 10 spans along the longitudinal direction between the front band member 31 and the back band member 41, and longitudinal end portions 10ea and 10eb of the absorbent main body 10 are respectively joined and fixed to the closest band members 31 and 41, thus forming a substantially H-like outer shape in a plan view. The diaper 1 in this state is folded one time at a folding position that is at a predetermined longitudinal position CL1 of the absorbent main body 10 (a longitudinal central position CL1 of the diaper 1), and the lateral end portions 31e and 41e of the band members 31 and 41, which face each other in the folded state, are joined to each other in the previously described side-seal sections SS. Accordingly, the band members 31 and 41 become connected to each other in a ring shape, thus obtaining the diaper 1 in the underpants-shaped state in which the waist opening BH and the pair of leg openings LH are formed as shown in FIG. 2.

The absorbent main body 10 has an approximately rectangular shape in a plan view in the unfolded state shown in FIG. 3. The longitudinal direction of the absorbent main body 10 conforms to the longitudinal direction of the diaper 1. Also, as shown in FIG. 4, the absorbent main body 10 includes: an absorbent body 11; a liquid-permeable top sheet 13 that covers the absorbent body 11 from the skin side and forms the skin-side surface of the absorbent main body 10; and a liquid-impermeable back sheet 15 that covers the absorbent body 11 from the non-skin side and forms the non-skin side of the absorbent main body 10.

The absorbent body 11 has a liquid-absorbent absorbent core 11c, and a core-wrapping sheet (not shown) that covers the outer circumferential surface of the core 11c. The absorbent core 11c is a molded body that is made of a predetermined liquid absorbent material such as pulp fiber or superabsorbent polymer, and is approximately hourglass-shaped in a plan view, as one example of a predetermined shape. The core-wrapping sheet can be made of a liquid-permeable sheet such as tissue paper or nonwoven fabric, but the core-wrapping sheet is not required to be provided. Also, the absorbent core 11c is not limited in any way to having the aforementioned approximately hourglass-like shape in a plan view, and may have another shape.

The top sheet 13 is a liquid-permeable soft sheet made of nonwoven fabric or the like. The back sheet 15 is also a liquid-impermeable soft sheet. One example of the back sheet 15 is a double-layer structure laminate sheet 15 including: a liquid-impermeable leak-proof sheet made of a polyethylene film (PE) or a polypropylene film; and an exterior sheet that is made of nonwoven fabric and is affixed to the non-skin side of the leak-proof sheet.

As shown in FIG. 3, at least the back sheet 15 is a sheet having a planar size according to which it projects from the absorbent body 11 in the longitudinal direction and the lateral direction. Leg gathers LG that stretch and contract in the longitudinal direction are formed in the portions that protrude in the lateral direction. Specifically, elastic strings 17 that serve as elastic members and extend in the longitudinal direction are fixed in the protruding portions in a state of being stretched in the longitudinal direction, thus forming the stretchable leg gathers LG in these portions.

Also, as shown in FIGS. 3 and 4, the absorbent main body 10 has barrier cuffs LSG as leak-proof wall portions in the lateral end portions for the purpose of preventing lateral leakage. Specifically, in the lateral end portions of the absorbent main body 10, a configurations are provided in which elastic strings 18 serving as elastic members 18 and extending in the longitudinal direction are attached, in a state of being stretched in the longitudinal direction, to sheet-like portions that will form the barrier cuffs LSG.

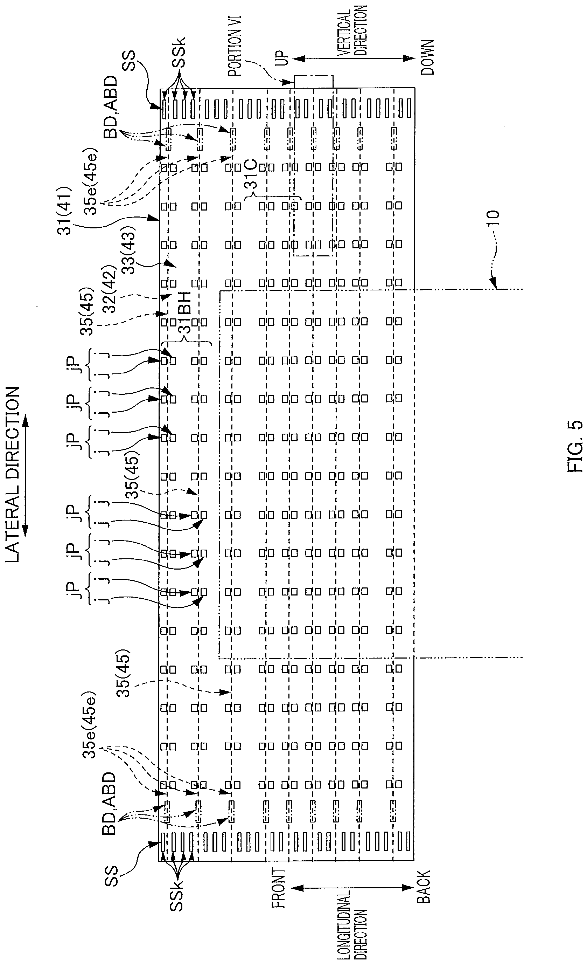

As shown in FIG. 3, the front band member 31 is a sheet-like member that is approximately rectangular in a plan view and is constituted by two nonwoven fabric sheets 32 and 33. Specifically, as shown in FIG. 4, the two nonwoven fabric sheets 32 and 33 are overlaid on each other in the thickness direction. And, the pair of facing surfaces that face each other are joined to each other by welded portions j (corresponding to joining portions) that are arranged discretely in the vertical direction (longitudinal direction) and the lateral direction as shown in later-described FIG. 5. As shown in FIG. 3, the front band member 31 is arranged so as to protrude out from the absorbent main body 10 on the two lateral sides, and is overlaid on and joined to the non-skin side of the front end portion 10ea of the absorbent main body 10.

Similarly to the front band member 31, the back band member 41 is also a sheet-like member that is approximately rectangular in a plan view and is constituted by two nonwoven fabric sheets 42 and 43. Specifically, as shown in FIG. 4, the two nonwoven fabric sheets 42 and 43 are overlaid on each other in the thickness direction, and, similarly to the front band member 31 shown in FIG. 5, the pair of facing surfaces that face each other are joined to each other by welded portions j (corresponding to joining portions) that are arranged discretely in the vertical direction (longitudinal direction) and the lateral direction. As shown in FIG. 3, the back band member 41 is arranged so as to protrude out from the absorbent main body 10 on the two lateral sides, and is overlaid on and joined to the non-skin side of the back end portion 10eb of the absorbent main body 10.

Note that when the content described below is the same for both the front band member 31 and the back band member 41, only the front band member 31 will be described as a representative for both, and corresponding portions of the back band member 41 are simply indicated in parentheses.

FIG. 5 is a schematic plan view of the front band member 31 in the unfolded state as viewed from the non-skin side.

As shown in FIG. 5, a plurality of elastic strings 35, 35... (45, 45...), which are elastic members that extend in the lateral direction, are inserted side-by-side in the vertical direction between the pair of mutually-opposing facing surfaces of the two nonwoven fabric sheets 32 and 33 (42 and 43) of the front band member 31 (41). And the elastic strings 35, 35... (45, 45...) are attached to the nonwoven fabric sheets 32 and 33 (42 and 43) with use of the welded portions j, j... that were mentioned above. Accordingly, the front band member 31 (41) is given stretchability in the lateral direction. Also, the previously mentioned welded portions j, j... not only have a function of joining the pair of facing surfaces of the two nonwoven fabrics 32 and 33 (42 and 43) to each other, but also have a function of attaching the elastic strings 35 (45) to the two nonwoven fabric sheets 32 and 33 (42 and 43).

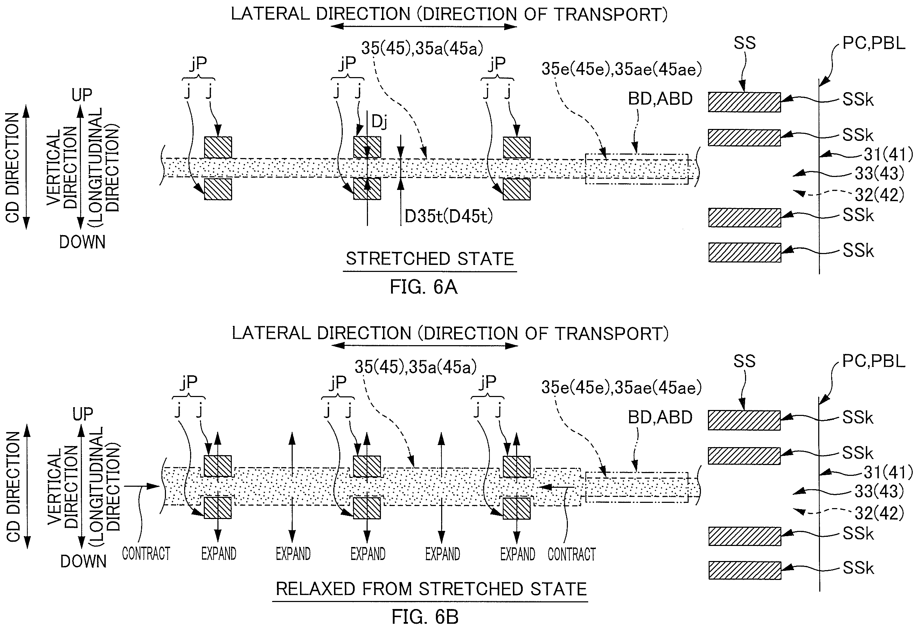

FIGS. 6A and 6B are schematic enlarged views of a portion VI in FIG. 5, and are for illustrating the latter function of the welded portions j, that is to say the function of attaching the elastic strings 35 (45).

As shown in FIG. 5, the welded portions j, j... are provided for each of the elastic strings 35 (45) that extend in the lateral direction. Also, the welded portions j are formed in pairs of portions on respective sides of the corresponding elastic string 35 in the vertical direction, that is to say, each pair of welded portions j that are side-by-side in the vertical direction make up welded portion pair jP. These welded portion pairs jP are formed side-by-side in the lateral direction spacing between welded portion pairs jP that are adjacent in the lateral direction. Also, as shown in FIG. 6A, the pair of welded portions j that form each welded portion pair jP are separated by a gap Dj in the vertical direction, and the size of this gap Dj is set the same as or somewhat larger than a vertical size D35t (D45t) of the elastic strings 35 (45) in a state of being stretched to a target stretch factor in the lateral direction. In the diaper 1 in the underpants-shaped state shown in FIG. 2, the elastic strings 35 (45) are relaxed from the state of being stretched to the aforementioned stretch factor. Accordingly, in this underpants-shaped state, as shown in FIG. 6B, the elastic strings 35 (45) contract in the lateral direction and expand in the vertical direction, and here, based on the size relationship described above, the expansion of the elastic strings 35 (45) in the vertical direction is restricted by the pairs of welded portions j. Accordingly, the elastic strings 35 (45) are substantially sandwiched and pressed in the vertical direction by the pairs of welded portions j, and as a result, the elastic strings 35 (45) are attached in the front band member 31 (41).

It should be noted that the strength of the attachment of the elastic strings 35 (45) with this method is substantially based on only the pressing between the welded portions j, and therefore is lower than the attachment strength in the case of being fixed by adhesion with a hot-melt adhesive. For this reason, as described at the beginning, there is a risk that the lateral end portions 35e (45e) of the elastic strings 35 (45) in the underpants-shaped state will separate from pairs of welded portions j.

In view of this, in this example, as shown in FIGS. 6A and 6B, in the lateral end portions 35e (45e) of the elastic strings 35 (45), the elastic strings 35 (45) are joined to the pair of mutually-opposing facing surfaces of the two nonwoven fabric sheets 32 and 33 (42 and 43) with use of hot-melt adhesive BD. This therefore suppresses the case where the lateral end portions 35e (45e) of the elastic strings 35 (45) separate from the nonwoven fabric sheets 32 and 33 (42 and 43) of the front band member 31 (41) .

It should be noted that the aforementioned stretch factor is a value R (=L1/ L0) that indicates the ratio of a stretched total length L1 of the elastic string 35 (45) to a total length L0 in a natural no-load state. The aforementioned target stretch factor is selected from the range of 1.5 to 4.0, for example.

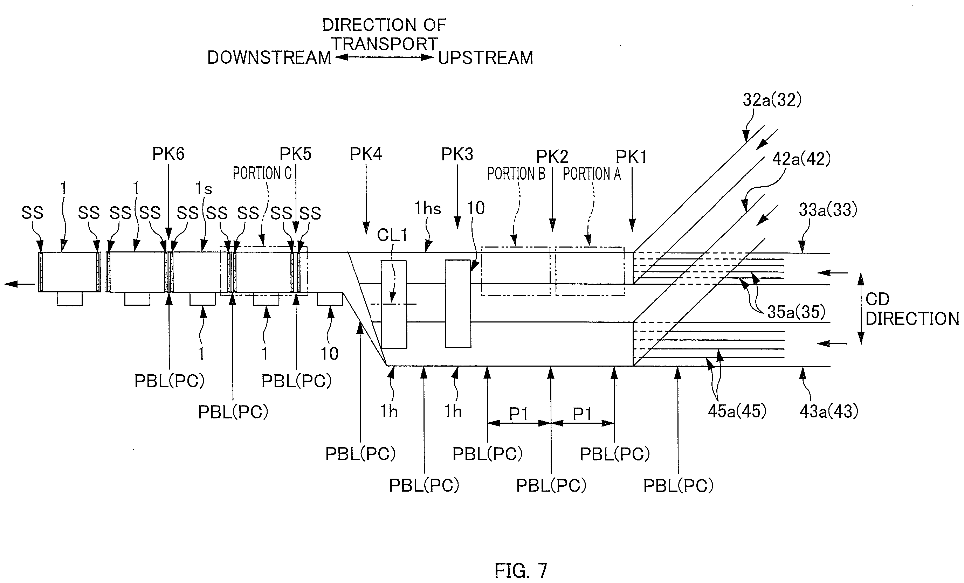

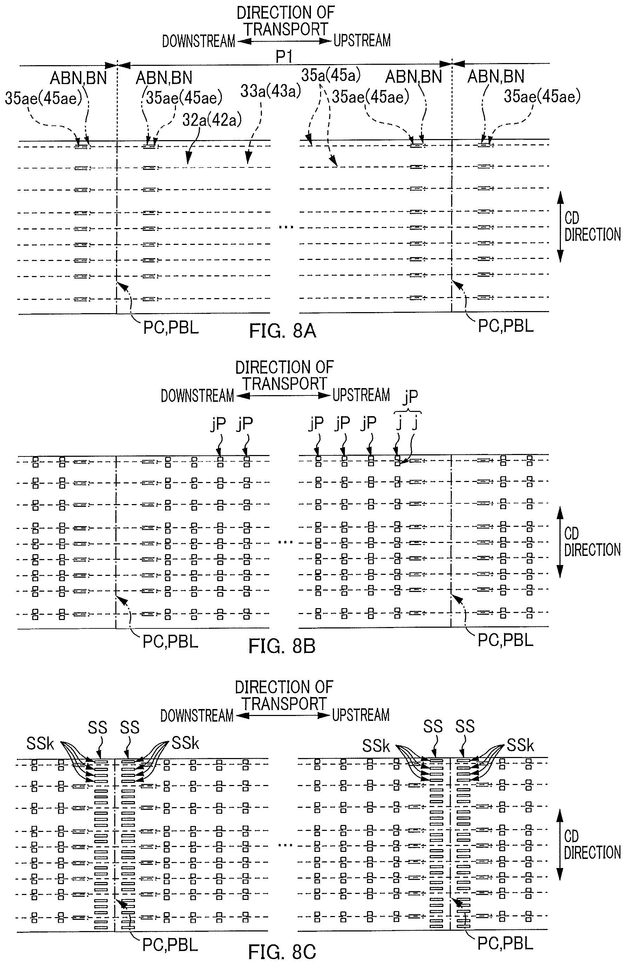

This diaper 1 is manufactured in a manufacturing line. FIG. 7 is a schematic plan view showing a partial perspective view of the manufacturing of the diaper 1 in the manufacturing line. Also, FIGS. 8A, 8B, and 8C are respectively schematic enlarged views of portions A, B, and C in FIG. 7.

In this manufacturing line, for example, the two nonwoven fabric sheets 32 and 33 pertaining to the front band member 31 are transported in the form of continuous sheets 32a and 33a that are continuous in the direction of transport, and likewise, the two nonwoven fabric sheets 42 and 43 pertaining to the back band member 41 are transported in the form of continuous sheets 42a and 43a that are continuous in the direction of transport. The continuous sheets 32a, 33a, 42a, and 43a pass through processing positions PK1 to PK6 that are set in the direction of transport, and the continuous sheets 32a, 33a, 42a, and 43a are subjected to processes that correspond to the processing positions PK1, PK2, and so on.

Note that here, the direction that is orthogonal to both the thickness direction and the direction of transport of the continuous sheets 32a, 33a, 42a, and 43a is defined as "CD direction", and in this example, the continuous sheets 32a, 33a, 42a, and 43a (i.e., the two continuous sheets 32a and 33a pertaining to the front band member 31 and the two continuous sheets 42a and 43a pertaining to the back band member 41) are transported side-by-side in the CD direction. Note that there is no limitation whatsoever to this.

Also, in this example, the processing positions at which processes are performed, namely the first to sixth processing positions PK1 to PK6, are set side-by-side in this order from upstream to downstream in the direction of transport. At the processing positions PK1, PK2, and so on, the processes that are performed on the continuous sheets 32a and 33a pertaining to the front band member 31 are substantially the same as those performed on the two continuous sheets 42a and 43a pertaining to the back band member 41.

For this reason, the front band member 31 and the back band member 41 will not be distinguished from each other when the same content applies hereinafter. For example, in the following description, the term "band member 31 (41)" will simply be used, or the term "the two continuous sheets 32a and 33a (42a and 43a)" will simply be used. Note that in such cases, in the terms indicating the members, such as "the continuous sheets 32a and 33a (42a and 43a)", "the elastic strings 35 (45)", and "the elastic-string continuous bodies 35a (45a) ", the reference signs that immediately follow the terms are the reference signs pertaining to the front band member 31, and the subsequent reference signs in the parentheses are the reference signs pertaining to the back band member 41.

As shown in FIG. 7, the two continuous sheets 32a and 33a (42a and 43a) pertaining to the band member 31 (41) are transported in a so-called lateral flow. Specifically, the two continuous sheets 32a and 33a (42a and 43a) are transported such that the direction corresponding to the lateral direction of the diaper 1 conforms to the direction of transport. For this reason, in the two continuous sheets 32a and 33a (42a and 43a), boundary positions PBL between two diapers 1 that are adjacent in the lateral direction are virtually set at a product pitch P1 in the direction of transport. Also, at the sixth processing position PK6 located at the end of the manufacturing line, the boundary position PBL is a cutting target position PC at which the two continuous sheets 32a and 33a (42a and 43a) are cut to produce a single-cut diaper 1.

Note that the two continuous sheets 32a and 33a (42a and 43a) pertaining to the band member 31 (41) are transported by appropriate transporting devices (not shown) such as a belt conveyor and transporting rollers. Accordingly, unless particularly stated otherwise, it is assumed that the two continuous sheets 32a and 33a (42a and 43a) are transported in the direction of transport by such transporting devices. Examples of the belt conveyor includes a normal belt conveyor that has an endless belt that is driven to rotate as a transporting surface, and a suction belt conveyor that has a function for suction to the outer circumferential surface of an endless belt.

The following is a detailed description of the diaper 1 manufacturing process.

As shown in FIG. 7, first, the two continuous sheets 32a and 33a (42a and 43a) pertaining to the band member 31 (41) pass the first processing position PK1. While passing, the two continuous sheets 32a and 33a (42a and 43a) are overlaid on each other in the thickness direction. At this time, the elastic-string continuous bodies 35a, 35a... (45a, 45a...) that are continuous in the direction of transport are inserted and arranged side-by-side in the CD direction between the two mutually-opposing facing surfaces of the two continuous sheets 32a and 33a (42a and 43a), in a state of being stretched in the direction of transport to the previously described target stretch factor. To portions of the elastic-string continuous bodies 35a, 35a... (45a, 45a...), the hot-melt adhesive BD has been applied in advance before the insertion. Specifically, the hot-melt adhesive BD has already been selectively applied to portions 35ae (45ae) of the elastic-string continuous bodies 35a, 35a... (45a, 45a...); the portions 35ae (45ae) are to be the end portions 35e (45e) of the elastic strings 35 (45) in the direction of transport when the elastic-string continuous bodies 35a, 35a... (45a, 45a...) are cut at the previously described cutting target position PC.

Accordingly, as shown in FIG. 8A, the portions 35ae (45ae) of the elastic-string continuous bodies 35a (45a) that are to be the end portions 35e (45e) are fixed between the pair of facing surfaces of the continuous sheets 32a and 33a (42a and 43a) by the adhesive BD, but the portions other than the portions 35ae (45ae) in the continuous bodies 35a (45a) are not fixed by the adhesive BD (corresponding to a fixing step). For this reason, the adhesive force of the adhesive BD can suppress the case where the end portions 35e (45e) of the elastic strings 35 (45) shown in FIGS. 5 and 6B become separated from the nonwoven fabric sheets 32 and 33 (42 and 43) of the band members 31 (41) when the elastic-string continuous bodies 35a, 35a... (45a, 45a...) are cut at the cutting target position PC together with the two continuous sheets 32a and 33a (42a and 43a) at the later-described sixth processing position PK6.

This application of the adhesive BD can be performed using an adhesive application device that is not shown, for example. The adhesive application device has a plurality of nozzles that discharge the adhesive BD, a pump that supplies the adhesive BD to the nozzles, and a valve for switching the state of the nozzles between a state of discharging the adhesive BD and a stopped state.

Also, the adhesive BD is not limited whatsoever to being applied to the elastic-string continuous bodies 35a (45a). Specifically, the adhesive may be applied to at least one of the two facing surfaces of the two continuous sheets 32a and 33a (42a and 43a) such that the elastic-string continuous bodies 35a (45a) are fixed to the at least one facing surface. Note that details of application regions ABD of the adhesive BD will be described later.

Next, as shown in FIG. 7, the two continuous sheets 32a and 33a (42a and 43a) pass the second processing position PK2. At this time, as shown in FIG. 8B, the previously described welded portions j, j... are formed as joining portions in the two continuous sheets 32a and 33a (42a and 43a), and thus the pair of facing surfaces of the two continuous sheets 32a and 33a (42a and 43a) are joined to each other by the welded portions j, j... (corresponding to a joining-portion forming step).

Here, as previously described, in this manufacturing line, the lateral direction of the diaper 1 conforms to the direction of transport, and the vertical direction of the diaper 1 conforms to the CD direction. For this reason, the welded portions j are formed in pairs, on two sides in the CD direction of the elastic-string continuous bodies 35a (45a). Specifically, as shown in FIG. 8B, two welded portions j that are side-by-side on respective sides in the CD direction of each continuous body 35a (45a) form a welded portion pair jP. These welded portion pairs jP are formed side-by-side in the direction of transport spacing between welded portion pairs jP that are adjacent in the direction of transport.

Also, as shown in FIG. 6A, the two welded portions j that form each welded portion pair jP are separated by the gap Dj in the CD direction, and here, the size of the gap Dj is the same as or somewhat larger than the size D35t (D45t) in the CD direction of the elastic-string continuous bodies 35a (45a) which are located at the second processing position PK2 and which are in a state of being stretched to the target stretch factor in the direction of transport.

Accordingly, when the elastic-string continuous bodies 35a (45a) are cut at the later-described sixth processing position PK6 thus relaxing the stretched state of the elastic strings 35 (45), as shown in FIG. 6B, the elastic strings 35 (45) contract in the direction of transport and attempt to expand in the CD direction. But, the elastic strings 35 (45) are sandwiched and pressed in the CD direction between the pair of welded portions j, and thus the elastic strings 35 (45) are attached to the two nonwoven fabric sheets 32 and 33 (42 and 43) of the band member 31 (41).

The welded portions j can be formed using a heat sealing device or an ultrasonic sealing device (corresponding to a joining-portion forming device) that is not shown, for example. A heat sealing device has a pair of rolls that are heated while rotating in the direction of transport, for example. One of the rolls is a heat emboss roll that has protrusion portions corresponding to the welded portions j on the outer circumferential surface, and the other roll is an anvil roll that receives the protrusion portions with a smooth outer circumferential surface. Also, an ultrasonic sealing device has a horn that has a vibrating surface that vibrates in a normal direction, and an anvil roll that rotates in the direction of transport, for example. The anvil roll has protrusion portions that correspond to the welded portions j on the outer circumferential surface in order to receive the vibrating surface.

Next, as shown in FIG. 7, the two continuous sheets 32a and 33a pertaining to the front band member 31 and the two continuous sheets 42a and 43a pertaining to the back band member 41 all pass the third processing position PK3. At this time, the single-cut absorbent main body 10 produced in a separate step (not shown) is fixed in a state of spanning between the two continuous sheets 32a and 33a pertaining to the front band member 31 and the two continuous sheets 42a and 43a pertaining to the back band member 41, thus forming the approximately ladder-shaped diaper continuous body 1hs in which approximately H-shaped unfolded diapers 1h, 1h... are continuous with each other.

The absorbent main body 10 is fixed using a rotating drum device that is not shown, for example. The rotating drum device has a rotating drum that rotates in the direction of transport, and the rotating drum has a plurality of holding portions that detachably hold the absorbent main body 10 to the outer circumferential surface, for example.

Next, the approximately ladder-shaped diaper continuous body 1hs passes the fourth processing position PK4. At this time, the absorbent main body 10 is folded one time at a predetermined position CL1 in the CD direction, and therefore the two continuous sheets 32a and 33a pertaining to the front band member 31 and the two continuous sheets 42a and 43a pertaining to the back band member 41 are overlaid on each other in the thickness direction.

The folding can be performed using a fold guiding device that is not shown, for example. The fold guiding device has a guide plate and guide rollers that are arranged at predetermined positions in the direction of transport, for example. The guide plate and the guide rollers guide the approximately ladder-shaped diaper continuous body 1hs passing at the arrangement position so as to be folded one time.

Next, the folded diaper continuous body 1hsb passes the fifth processing position PK5. At this time, concerning the two continuous sheets 32a and 33a pertaining to the front band member 31 and concerning the two continuous sheets 42a and 43a pertaining to the back band member 41, the continuous sheets 32a and 33a and 42a and 43a which are overlaid in the thickness direction are welded at positions on two sides of the cutting target position PC in the direction of transport so as to form a pair of side-seal sections SS (corresponding to a welding step), thus fixing the diaper continuous body 1hsb in the folded state. This consequently produces a pull-on diaper continuous body 1s in which a plurality of pull-on diapers 1, 1... are connected in the lateral direction.

Here, as shown in FIG. 8C, the side-seal sections SS have a plurality of welded portions SSk, SSk... that are side-by-side in the CD direction (vertical direction) . The welded portions SSk weld together the continuous sheet 32a of the front band member 31 and the continuous sheet 42a of the back band member 41, weld together the pair of facing surfaces of the continuous sheets 32a and 33a of the front band member 31, and weld together the pair of facing surfaces of the continuous sheets 42a and 43a of the back band member 41.

Also, in this example, the planar shape of the welded portions SSk is a laterally elongated rectangular shape that is longer in the lateral direction, which is the direction of transport, than in the vertical direction, which is the CD direction. Note that there is no limitation whatsoever to this. For example, it may have a parallelogram shape or oval shape, or may have another shape . Also, in the example in FIG. 8C, the longitudinal direction of the welded portions SSk conforms to the lateral direction, which is the direction of transport, but there is no limitation whatsoever to this. In other words, the longitudinal direction of the welded portions SSk may conform to the vertical direction, which is the CD direction, or may conform to a direction that intersects both the lateral direction and the vertical direction.

The side-seal sections SS can be formed using a heat sealing device or an ultrasonic sealing device that is not shown, for example. A heat sealing device has a pair of rolls that are heated while rotating in the direction of transport, for example. One of the rolls is a heat emboss roll that has protrusion portions corresponding to the welded portions SSk of the side-seal sections SS on the outer circumferential surface, and the other roll is an anvil roll that receives the protrusion portions with a smooth outer circumferential surface. Also, an ultrasonic sealing device has a horn that has a vibrating surface that vibrates in a normal direction, and an anvil roll that rotates in the direction of transport, for example. The anvil roll has protrusion portions that correspond to the welded portions SSk on the outer circumferential surface in order to receive the vibrating surface.

Next, as shown in FIG. 7, the pull-on diaper continuous body 1s passes the sixth processing position PK6. At this time, the continuous body 1s is cut at the cutting target position PC that is located between a pair of side-seal sections SS (corresponding to a cutting step), thus obtaining the diaper 1.

Note that at the time of this cutting, the two continuous sheets 32a and 33a (42a and 43a) pertaining to the front band member 31 and the back band member 41 and the elastic-string continuous bodies 35a, 35a... (45a, 45a...) are cut at the cutting target position PC. Due to the corresponding relaxation of the stretched state of the elastic strings 35 (45), the elastic strings 35 (45) are sandwiched and pressed by the pairs of welded portions j in the welded portion pairs jP and are thus attached to the band members 31 and 41 as in the description of the third processing position PK3. Also, due to the attachment strength based on the pressing between welded portions j, there is a risk of separation of the end portions 35e (45e) of the elastic strings 35 (45) from the pairs of welded portions j. Therefore, in the present embodiment, the portions 35ae (45ae) of the elastic-string continuous bodies 35a (45a) corresponding to the end portions 35e (45e) are adhered and fixed to the pair of facing surfaces of the two continuous sheets 32a and 33a (42a and 43a) with the adhesive BD as in the description of the first processing position PK1.

This cutting can be performed using a cutter device (corresponding to a cutting device) that is not shown, for example. A cutter device has a pair of rolls that rotate in the direction of transport, for example. One of the rolls is a cutter roll that has a cutter blade on the outer circumferential surface, and the other roll is an anvil roll having an outer circumferential surface that receives the cutter blade.

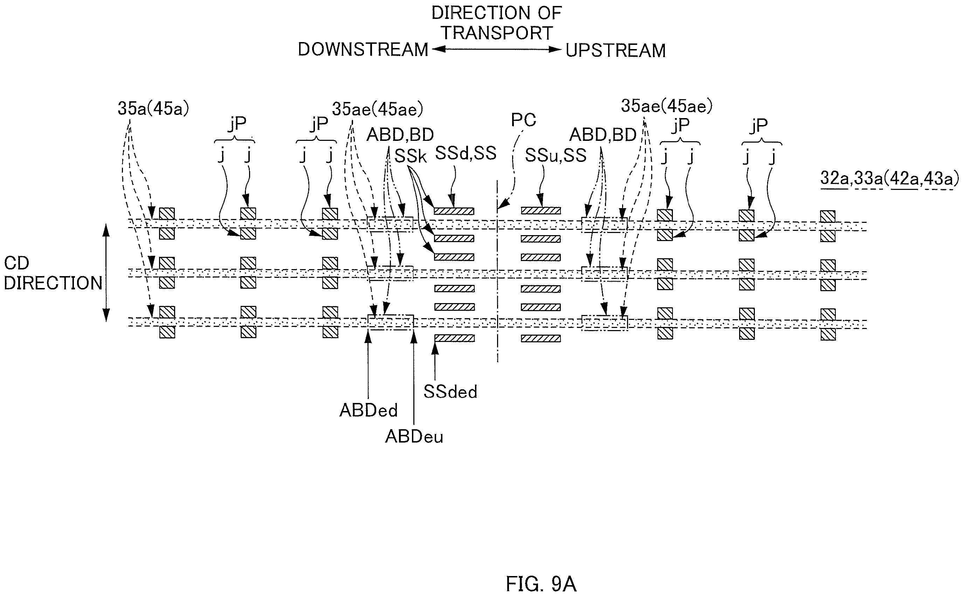

FIGS. 9A to 10C are illustrative diagrams of variations of application regions ABD of the adhesive BD for fixing the end portions 35e (45e) of the elastic strings 35 (45). Note that all of these figures are schematic plan views of a portion in the vicinity of the cutting target position PC in the pull-on diaper continuous body 1s before being cut in the sixth processing position PK6, as viewed in the thickness direction.

Also, the content described below is the same for both the elastic-string continuous bodies 35a pertaining to the front band member 31 and the elastic-string continuous bodies 45a pertaining to the back band member 41. For this reason, only the former will be described as a representative for both, the latter will only be indicated by corresponding member reference signs in parentheses in the figures, and descriptions will not be given for the latter.

As previously described with reference to FIGS. 6A and 6B, when the diaper continuous body 1s is cut at the cutting target position PC, the two end portions 35e are formed in each elastic string 35 on respective sides of the cutting target position PC in the direction of transport. Accordingly, the portions 35ae of the elastic-string continuous bodies 35a, which are to be the end portions 35e of the elastic strings 35, are located on two sides of the cutting target position PC in the direction of transport. Also, the adhesive BD is for fixing the portions 35ae to the pair of facing surfaces of the continuous sheets 32a and 33a. For this reason, in all of the examples in FIGS. 9A to 10C, the application regions ABD of the adhesive BD are formed on two sides of the cutting target position PC in the direction of transport.

Note that the application regions ABD on the two sides are formed so as to be approximately in line symmetry with each other with respect to the cutting target position PC. For this reason, only the application region ABD located downstream with respect to the cutting target position PC in the direction of transport will be described hereinafter, and the application region ABD located upstream will not be described.

Also, in the following description, out of the two side-seal sections SS that are formed on two sides in the direction of transport with respect to the cutting target position PC, the side-seal section SS located downstream in the direction of transport will also be referred to as a "downstream side-seal section SSd", and the side-seal section SS located upstream will also be referred to as an "upstream side-seal section SSu".

The following describes variations of the application regions ABD of the adhesive BD with reference to FIGS. 9A to 10C, and here, in each of the examples in FIGS. 9A to 10C, one type of application region ABD pattern is applied to all of the elastic-string continuous bodies 35a pertaining to the front band member 31. In other words, the pattern of application regions ABD is the same for all of the elastic-string continuous bodies 35a, 35a... here. However, there is no limitation whatsoever to this.

For example, the pattern of the application regions ABD that is applied to the elastic-string continuous bodies 35a pertaining to the front band member 31 may be selected from among the variations in FIGS. 9A to 10C for each of the elastic-string continuous bodies 35a. According to this configuration, actions and effects unique to each applied application region ABD pattern can be achieved for each of the elastic-string continuous bodies 35a.

More specifically, in the examples shown in FIGS. 9A to 10C, the application regions ABD of the adhesive BD are set for all of the elastic-string continuous bodies 35a of the front band member 31, thus suppressing the previously described problem for all of the elastic-string continuous bodies 35a, that is to say the problem of separation of the end portions 35e of the elastic strings 35. However, there is no limitation whatsoever to this.

For example, if the application regions ABD are set for at least one of all of the elastic-string continuous bodies 35a pertaining to the front band member 31, desirably one-third or more, more desirably half or more, and further desirably two-thirds or more of the elastic-string continuous bodies 35a, then the aforementioned problem can be suppressed for at least the elastic-string continuous bodies 35a for which the application regions ABD have been set. For this reason, elastic-string continuous bodies 35a for which the application regions ABD are not set may exist among all of the elastic-string continuous bodies 35a pertaining to the front band member 31.

First, in the examples shown in FIGS. 9A to 9D, downstream ends ABDed of the application regions ABD are located downstream in the direction of transport with respect to a downstream end SSded of the downstream side-seal section SSd.

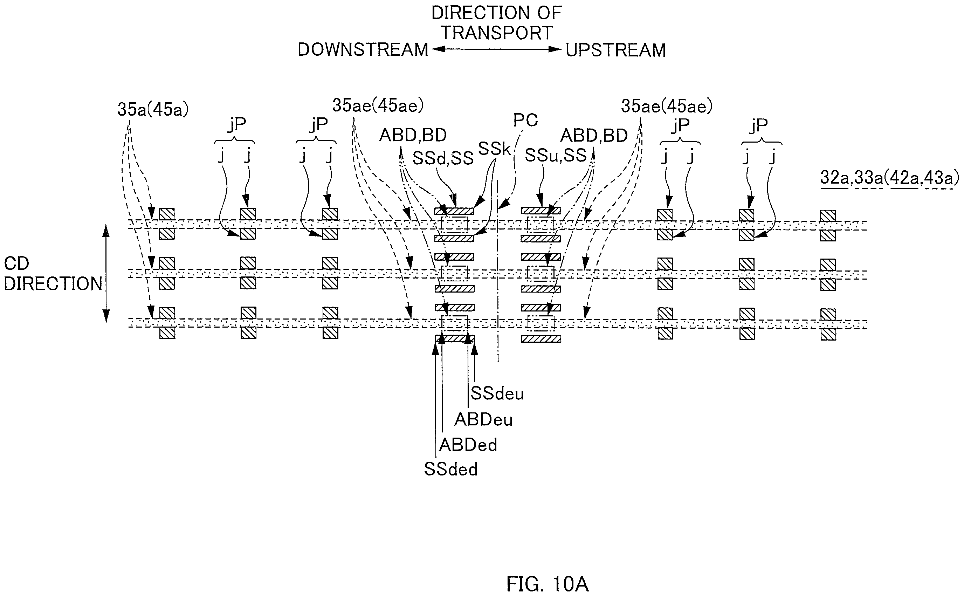

Accordingly, a larger size in the direction of transport can be ensured for the application regions ABD compared to the case where the downstream ends ABDed of the application regions ABD are located upstream with respect to the downstream end SSded of the downstream side-seal section SSd (e.g., the example in FIG. 10A). Accordingly, the end portions 35e of the elastic strings 35 can be fixed more firmly by the adhesive, and thus making it possible to reliably suppress the case where the end portions 35e become separated from the front band member 31.

Also, in the example shown in FIG. 9A, upstream ends ABDeu of the application regions ABD are located downstream in the direction of transport with respect to the downstream end SSded of the downstream side-seal section SSd.

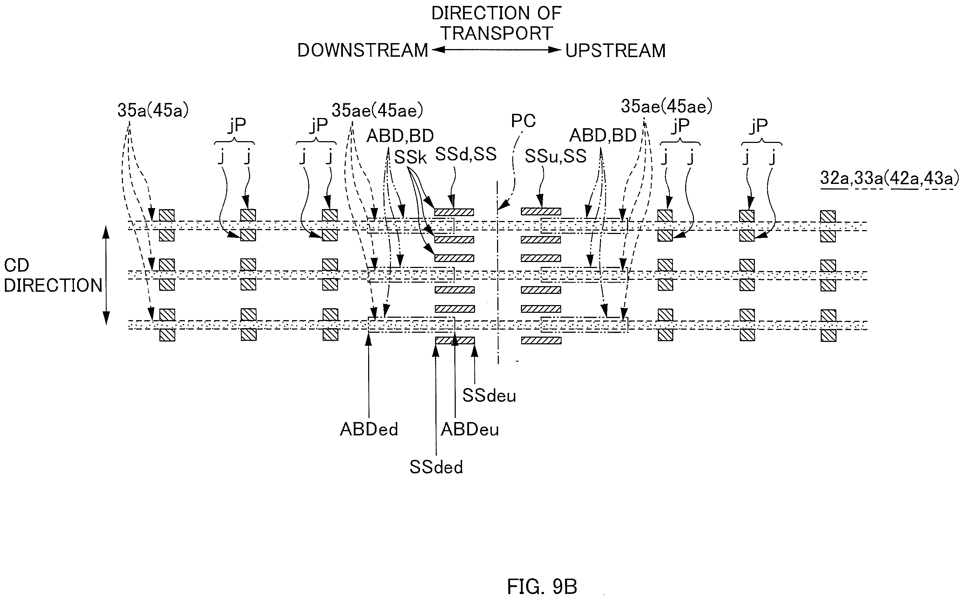

Accordingly, a smaller size in the direction of transport (lateral direction) can be set for the application regions ABD compared to the case where the upstream ends ABDeu of the application regions ABD are located upstream with respect to the downstream end SSded of the downstream side-seal section SSd (e.g., the examples in FIGS. 9B to 9D). Accordingly, it is possible to prevent a problem that can occur if the application regions ABD are too large, such as the stretchability of the elastic strings 35 or the softness of the front band member 31 being impaired by hardening of the adhesive BD.

Also, in the example shown in FIG. 9B, the upstream ends ABDeu of the application regions ABD are located in the direction of transport between the downstream end SSded and the upstream end SSdeu of the downstream side-seal section SSd.

Accordingly, a larger size in the direction of transport can be ensured for the application regions ABD compared to the case where the upstream ends ABDeu of the application regions ABD are located downstream with respect to the downstream end SSded of the downstream side-seal section SSd (e.g. , the example in FIG. 9A). Accordingly, the end portions 35e of the elastic strings 35 can be fixed more firmly by the adhesive BD, and thus making it possible to reliably suppress the case where the end portions 35e become separated from the front band member 31.

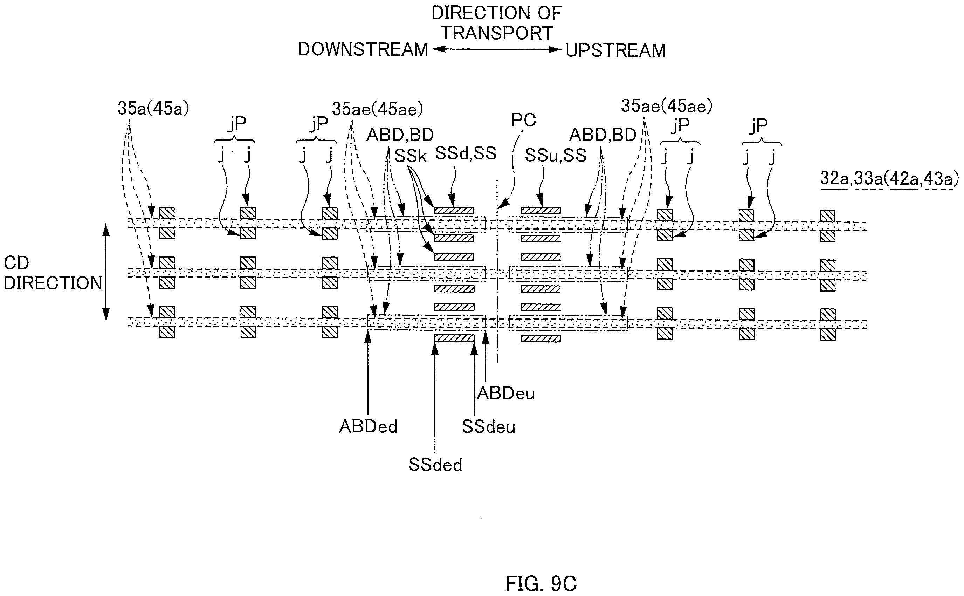

Also, in the example shown in FIG. 9C, the upstream ends ABDeu of the application regions ABD are located in the direction of transport between the upstream end SSdeu of the downstream side-seal section SSd and the cutting target position PC.

Accordingly, a larger size in the direction of transport can be ensured for the application regions ABD compared to the case where the upstream ends ABDeu of the application regions ABD are located downstream with respect to the upstream end SSdeu of the downstream side-seal section SSd (e.g., the examples in FIGS. 9A and 9B). Accordingly, the end portions 35e of the elastic strings 35 can be fixed more firmly by the adhesive BD, thus making it possible to reliably suppress the case where the end portions 35e become separated from the front band member 31.

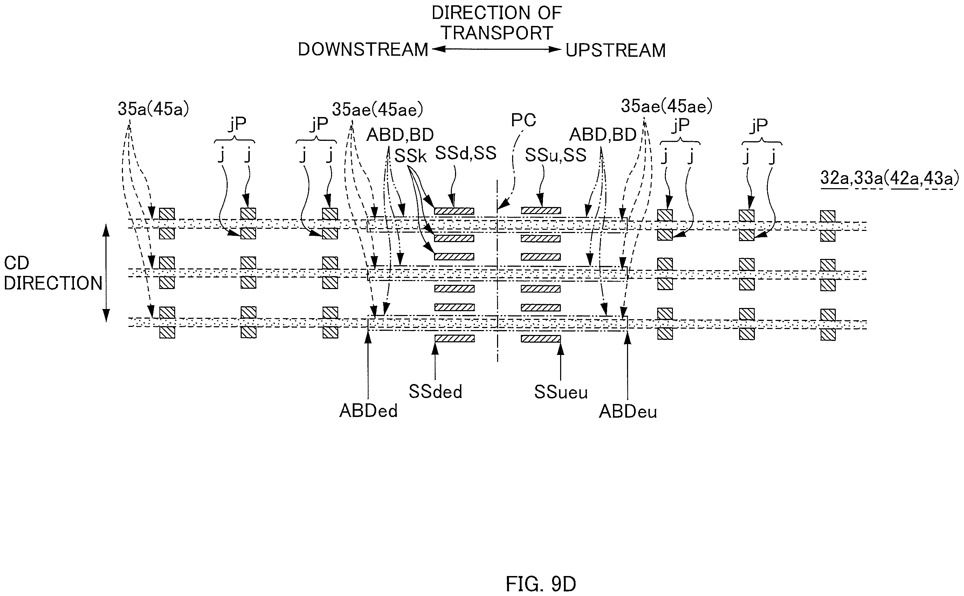

Also, in the example shown in FIG. 9D, the upstream ends ABDeu of the application regions ABD are located upstream in the direction of transport with respect to the cutting target position PC.

Accordingly, in the front band member 31, the size of the application regions ABD on the upstream side can be maximized, thus making it possible for the end portions 35e of the elastic strings 35 to be more firmly fixed by the adhesive BD.

It should be noted that in the case of the example shown in FIG. 9D, the application regions ABD extend beyond the upstream end SSueu of the upstream side-seal section SSu and further upstream in the direction of transport. Concerning these portions of the application regions ABD that are located upstream with respect to the cutting target position PC in the direction of transport, the portions contribute to fixing to the front band member 31 the end portions 35e of the elastic strings 35 of the diaper 1 having the upstream side-seal section SSu.

On the other hand, in the examples shown in FIGS. 10A to 10C, the downstream ends ABDed of the application regions ABD are located upstream in the direction of transport with respect to the downstream end SSded of the downstream side-seal section SSd.

Accordingly, the application regions ABD can be overall set closer to the cutting target position PC compared to the case where the downstream ends ABDed of the application regions ABD are located downstream with respect to the downstream end SSded of the downstream side-seal section SSd (e.g., the examples shown in FIGS. 9A to 9D) . Accordingly, the portions in which the stretchability of the elastic strings 35 is impaired by hardening of the adhesive BD can be positioned closer to the cutting target position PC, thus making it possible to prevent the portions in which the stretchability of the elastic strings 35 is impaired by hardening of the adhesive BD from becoming noticeable.

Also, in the example shown in FIG. 10A, the upstream ends ABDeu of the application regions ABD are located downstream in the direction of transport with respect to the upstream end SSdeu of the downstream side-seal section SSd.

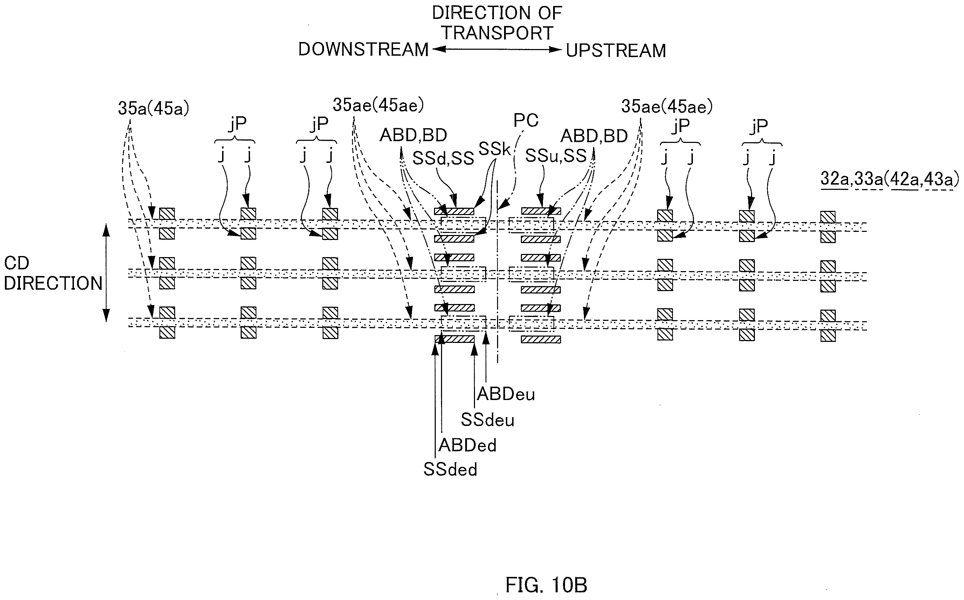

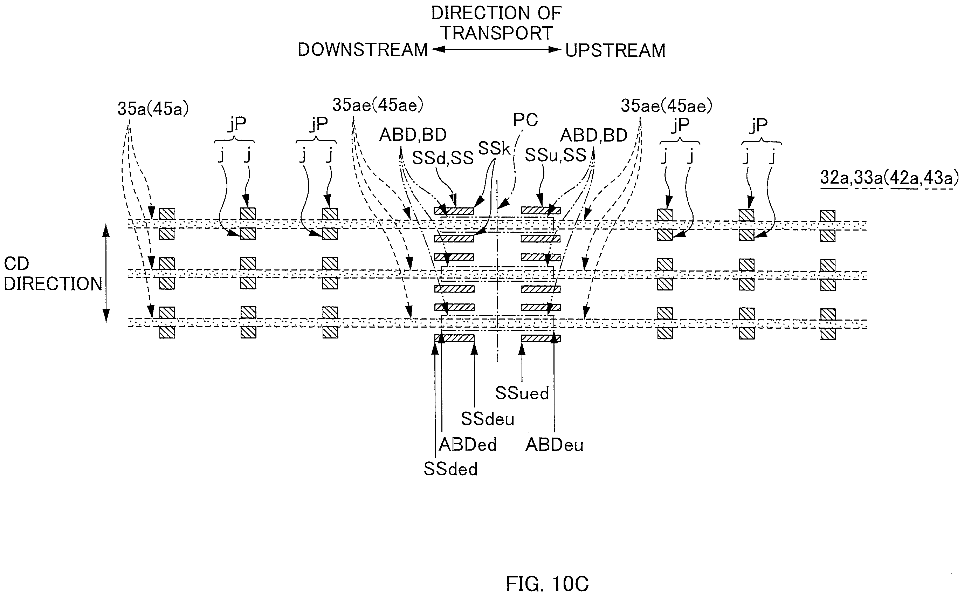

Accordingly, a smaller size in the direction of transport can be set for the application regions ABD compared to the case where the upstream ends ABDeu of the application regions ABD are located upstream with respect to the upstream end SSdeu of the downstream side-seal section SSd (e.g., the examples in FIGS. 10B and 10C). Accordingly, it is possible to prevent a problem that can occur if the application regions ABD are too large, such as the stretchability of the elastic strings 35 or the softness of the front band member 31 being impaired by hardening of the adhesive BD.

Also, in the example shown in FIG. 10B, the upstream ends ABDeu of the application regions ABD are located in the direction of transport between the upstream end SSdeu of the downstream side-seal section SSd and the cutting target position PC.

Accordingly, a larger size in the direction of transport can be ensured for the application regions ABD compared to the case where the upstream ends ABDeu of the application regions ABD are located downstream with respect to the upstream end SSdeu of the downstream side-seal section SSd (e.g., the example in FIG. 10A). Accordingly, the end portions 35e of the elastic strings 35 can be fixed more firmly by the adhesive BD, thus making it possible to reliably suppress the case where the end portions 35e become separated from the front band member 31.

Also, in the example shown in FIG. 10C, the upstream ends ABDeu of the application regions ABD are located upstream in the direction of transport with respect to the cutting target position PC.

Accordingly, in the front band member 31, the size of the application regions ABD on the upstream side can be maximized, thus making it possible for the end portions 35e of the elastic strings 35 to be more firmly fixed by the adhesive BD.

It should be noted that in the case of the example shown in FIG. 10C, the application regions ABD extend beyond the downstream end SSued of the upstream side-seal section SSu and further upstream in the direction of transport. Concerning these portions of the application regions ABD that are located upstream with respect to the cutting target position PC in the direction of transport, these portions contribute to fixing to the front band member 31 the end portions 35e of the elastic strings 35 of the diaper 1 having the upstream side-seal section SSu.

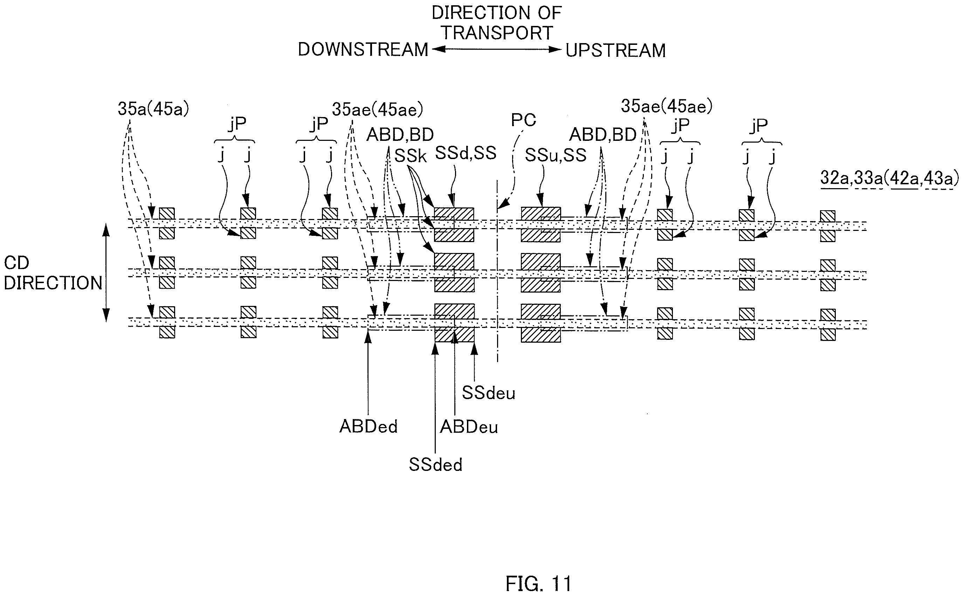

Also, in all of the examples in FIGS. 9B to 10C, it is desirable that the welded portions SSk of the downstream side-seal section SSd are provided so as to be overlapped with the application regions ABD as the diaper continuous body 1s (corresponding to the sheet-like member continuous body) is viewed in the thickness direction. For example, using the example in FIG. 9B as a representative of the examples in FIGS. 9B to 10C, as shown in FIG. 11, the welded portions SSk of the downstream side-seal section SSd may be provided so as to be overlapped with the application regions ABD.

According to this configuration, the welded portions SSk of the downstream side-seal section SSd can also contribute to fixing the end portions 35e of the elastic strings 35 to the front band member 31 (41) by the adhesive BD, thus improving the fixed strength of the end portions 35e of the elastic strings 35.

It should be noted that this overlapping can be realized by increasing, in the CD direction, the size of the welded portions SSk of the downstream side-seal section SSd for example, as can be understood from a comparison between FIG. 11 and FIG. 9B.

Also, in this case, from the viewpoint of reliable overlapping of the application regions ABD and the welded portions SSk, it is desirable that in the manufacturing line the adhesive BD is applied to at least one of the continuous sheets 32a and 33a and not to the elastic-string continuous bodies 35a.

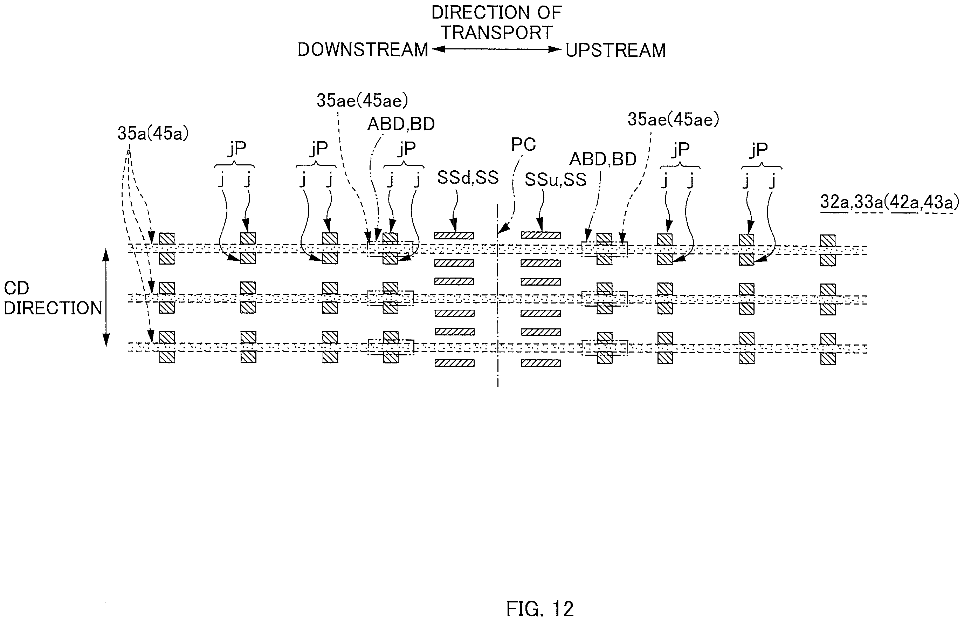

On the other hand, for all of the examples in FIGS. 9A to 9D as well, it is desirable that at least one of the welded portions j of the diaper continuous body 1s is provided so as to be overlapped with the application regions ABD as the diaper continuous body 1s is viewed in the thickness direction. For example, using the example in FIG. 9A as a representative for the examples in FIGS. 9A to 9D, as shown in FIG. 12, welded portion pairs jP may be added so as to be overlapped with the application regions ABD.

According to this configuration, pressing between the welded portions j can also contribute to the fixing of the end portions 35e of the elastic strings 35 to the front band member 31 by the adhesive BD, thus improving the fixed strength of the end portions 35e of the elastic strings 35.

Note that in this case, from the viewpoint of reliable overlapping of the application regions ABD and the welded portions j, it is desirable that in the manufacturing line the adhesive BD is applied to at least one of the continuous sheets 32a and 33a and not to the elastic-string continuous bodies 35a.

It should be noted that the thickness of the elastic strings 35 and the target stretch factor are sometimes the same for all of the elastic strings 35 provided in the front band member 31, and are sometimes not the same. For example, as shown in FIG. 5, in a portion 31BH of the front band member 31 that is near the waist opening BH in the vertical direction, from the viewpoint of reliable contact between the portion 31BH and the wearer, relatively thick elastic strings 35 (corresponding to first elastic members, and also called first elastic strings 35 hereinafter) are used in the portion 31BH, and the target stretch factor is also set to a higher value, thus increasing the tension (N) of the continuous bodies for the first elastic strings 35. In contrast, as shown in FIG. 5 for example, in a central portion 31C of the front band member 31 with respect to the vertical direction, from the viewpoint of loose contact between the portion 31C and the wearer, thinner elastic strings 35 (corresponding to second elastic members, and also called second elastic strings 35 hereinafter) that are thinner than the first elastic strings 35 are used in the portion 31C, and the target stretch factor is also set smaller than that of the first elastic strings 35, and therefore the tension (N) of the continuous bodies 35a of the second elastic strings is smaller than the tension (N) of the continuous bodies 35a of the first elastic strings.

Under such conditions, if the sizes of the application regions ABD in the direction of transport were set the same for both, this could lead to the problem of antinomy as described below. Specifically, if the application regions ABD are set to a size suited to the first elastic strings 35 that have higher tension, more adhesive BD than necessary is used for the second elastic strings 35 that have lower tension, and this consequently can lead to the problems of an increase in cost and impairing stretchability. However, if the application regions ABD are set to a size suited to the second elastic strings 35 that have lower tension, less adhesive BD than necessary is used for the first elastic strings 35 that have higher tension, and this consequently can lead to the problems of not being able to firmly fix the end portions 35e of the elastic strings 35, and thus the end portions 35e become separated.

For this reason, it is desirable that the size of the application regions ABD in the direction of transport is changed according to the tension. Specifically, it is desirable that the size of the application regions ABD of the adhesive BD in the direction of transport for the first elastic-string continuous bodies 35a that have higher tension is set higher than the size of the application regions ABD of the adhesive BD in the direction of transport for the second elastic-string continuous bodies 35a that have lower tension. For example, in the case of using the application region ABD pattern shown in FIG. 9A to the second elastic-string continuous bodies 35a that have lower tension, it is desirable that any of the application region ABD patterns shown in FIGS. 9B to 9D is used for the first elastic-string continuous bodies 35a that have higher tension.

### Other embodiments

Although the embodiment of the present disclosure have been described hereinabove, the above embodiment of the present disclosure is simply to facilitate understanding of the present disclosure and are not in any way to be construed as limiting the present disclosure. The present disclosure may variously be changed or altered without departing from its gist and encompass equivalents thereof. For example, modifications which will be described below are possible.

In the above embodiment, the welded portion j is illustrated as an example of the joining portion that joins the pair of facing surfaces to each other as shown in FIG. 5, but there is no limitation whatsoever to this. For example, the joining portion may be formed using an adhesive, and in this case, the adhesive is selectively applied, on at least one of the two facing surfaces, to a target formation position at which the joining portion is to be formed.

In the above embodiment, the method of the present invention is used to attach the elastic strings 35 serving as elastic members to the front band member 31 that is a sheet-like member as shown in FIG. 5, but there is no limitation whatsoever to this. For example, the method of the present invention may be used, for forming the barrier cuffs LSG in FIG. 3, when the elastic members 18 extending in the vertical direction are to be attached to the sheet-like portions for the barrier cuffs LSG. Or the method of the present invention may be used when the elastic members 17 (e.g., elastic strings) are to be attached to the portions of the absorbent main body 10 that are to become the leg gathers LG.

In the above embodiment, an example is described in which the sheet-like member continuous body has a configuration having the two continuous sheets 32a and 33a (42a and 43a) as shown in FIG. 7, but there is no limitation whatsoever to this. For example, a configuration is possible in which the pair of facing surfaces are formed by folding one continuous sheet at a predetermined position in the CD direction, and then inserting the elastic-string continuous bodies 35a (45a) between the pair of facing surfaces.

In the above embodiment, the elastic strings 35 (45) are illustrated as examples of elastic members, and the elastic-string continuous bodies 35a (45a) are illustrated as examples of the elastic-member continuous bodies. Spandex or the like are another specific example of such elastic strings 35 (45), and LYCRA (registered trademark) is an example of such a product. Also, the elastic strings 35 (45) can have a fiber density of 400 dtex to 1,000 dtex, for example.

In the above embodiment, the elastic strings 35 (45) are illustrated as examples of elastic members, and the elastic-string continuous bodies 35a (45a) are illustrated as examples of the elastic-member continuous bodies, but there is no limitation whatsoever to this. For example, rubber strips may be used as the elastic members, and rubber strip continuous bodies may be used as the elastic-member continuous bodies.

In the above embodiment, as shown in FIG. 5, all of the elastic strings 35, 35... provided in the front band member 31 are arranged so as to be continuous over substantially the entire length in the lateral direction, but there is no limitation whatsoever to this. For example, several of the elastic strings 35, 35... may be discontinuous at a central position in the lateral direction. Note that the same applies to the back band member 41 as well.

In the above embodiment, as shown in FIG. 3, the three-piece type of disposable diaper 1 is illustrated as an example of the absorbent article, but there is no limitation whatsoever to this. For example, the method of the present invention may be used when attaching elastic members such as elastic strings to a sheet-like member for use in a two-piece type of disposable diaper. A two-piece type disposable diaper is a type of diaper that has a first component and a second component: the first component is an exterior sheet having a two-layer structure and including a front portion, a crotch portion, and a back portion; and the second component is the absorbent main body 10 fixed to the skin-side surface of the exterior sheet. Also, the method of the present invention may be used when attaching elastic members such as elastic strings to a sheet-like member for use in a so-called tape-type disposable diaper. Note that a tape-type disposable diaper is a type of diaper that uses fastening tape in order to connect a front portion, which covers the stomach side of the wearer's trunk, to the back portion, which covers the back side of the wearer's trunk. Furthermore, the absorbent article is not limited whatsoever to being the disposable diaper 1. Specifically, the method of the present invention is applicable to any absorbent article that uses a sheet-like member to which elastic members such as those described above are attached. For this reason, the concept of this absorbent article also encompasses a urine absorbing pad, a sanitary napkin, and the like.

In the above embodiment, the welded portion j having an approximately square shape in a plan view is illustrated as an example of the joining portion as shown in FIG. 6, but the shape of the welded portion j is not limited in any way to this. For example, a circular shape may be applied, or an elongated shape having a longitudinal direction, such as a rectangular or oval shape, may be used. Note that in the latter case of a shape having a longitudinal direction, the longitudinal direction may conform to the direction of transport (lateral direction), may conform to the CD direction (vertical direction), or may conform to a direction that intersects both the direction of transport and the CD direction.

In the above embodiment, as shown in FIG. 5, the welded portions j, j... are provided in a so-called ladder-like arrangement that is defined in the lateral direction and the vertical direction (longitudinal direction) . Specifically, the welded portions j, j ... are provided at intersections between virtual lines extending in the lateral direction and virtual lines extending in the vertical direction, but there is no limitation whatsoever to this. For example, the welded portions j, j... may be provided in a so-called staggered arrangement by being providing at positions that are shifted in the lateral direction from the aforementioned intersections. Also, although the welded portions j, j... are arranged side-by-side in the vertical direction in FIG. 5, there is no limitation whatsoever to this. For example, the welded portions j, j... may be arranged side-by-side in a diagonal direction that intersects both the vertical direction and the lateral direction.

In the above embodiment, as shown in FIG. 5, a welded portion j is not provided between two welded portion pairs j P that are adj acent in the vertical direction (CD direction), but there is no limitation whatsoever to this. For example, one or more welded portions j may be provided between two welded portion pairs jP. Note that such a welded portion j does not contribute to attachment of the elastic strings 35 (45) to the nonwoven fabric sheets 32 and 33 (42 and 43), and only contributes to the joining of the nonwoven fabric sheets 32 and 33 (42 and 43) to each other.

### [Reference Signs List]

1 disposable diaper (absorbent article),
1hs approximately ladder-shaped diaper continuous body,
1hsb folded diaper continuous body,
1s pull-on diaper continuous body,
10 absorbent main body, 10ea end portion, 10eb end portion,
11 absorbent body, 11c absorbent core,
13 top sheet, 15 back sheet,
17 elastic string (elastic member), 18 elastic string (elastic member),
31 front band member (sheet-like member), 31e end portion,
31BH portion, 31C portion,
32 nonwoven fabric, 32a continuous sheet,
33 nonwoven fabric, 33a continuous sheet,
35 elastic string (elastic member), 35e end portion,
35a elastic-string continuous body (elastic-member continuous body), 35ae portion to be end portion,
41 back band member, 41e end portion,
42 nonwoven fabric, 42a continuous sheet,
43 nonwoven fabric, 43a continuous sheet,
45 elastic string (elastic member), 45e end portion,
45a elastic-string continuous body (elastic-member continuous body), 45ae portion to be end portion,
BH waist opening, LH leg opening,
LG leg gather, LSG barrier cuff,
BD hot-melt adhesive (adhesive),
ABD application region, ABDeu upstream end, ABDed downstream end, j welded portion (joining portion), jP welded portion pair,
PC cutting target position, PBL boundary position,
SS side-seal section,
SSu upstream side-seal section, SSueu upstream end, SSued downstream end,
SSd downstream side-seal section, SSdeu upstream end, SSded downstream end,
SSk welded portion,
CL1 central position (predetermined position),
PK1 first processing position, PK2 second processing position, PK3 third processing position,
PK4 fourth processing position, PK5 fifth processing position, PK6 sixth processing position

## Claims

1. A method for manufacturing a sheet-like member for an absorbent article,
the sheet-like member having an elastic member attached to the sheet-like member,
the sheet-like member being manufactured by inserting an elastic-member continuous body between a pair of mutually-opposing facing surfaces of a sheet-like member continuous body, and then cutting the sheet-like member continuous body at a cutting target position in a direction of transport of the sheet-like member continuous body,
the elastic-member continuous body being continuous in the direction of transport,
the sheet-like member continuous body being continuous in the direction of transport,
the method comprising:
a fixing step of fixing the elastic-member continuous body to at least one facing surface out of the pair of facing surfaces with use of an adhesive,
the elastic-member continuous body being stretched in the direction of transport,
the fixing step including fixing a portion of the elastic-member continuous body to the at least one facing surface with use of the adhesive,
the portion being a portion that is to be an end portion of the elastic member in the direction of transport when cutting the elastic-member continuous body at the cutting target position;
a joining-portion forming step of forming a plurality of joining portions spacing in the direction of transport,
the joining portions joining the pair of facing surfaces to each other,
the joining-portion forming step including forming the plurality of joining portions at positions on two sides of the elastic-member continuous body in a CD direction that intersects the direction of transport; and
a cutting step of producing the sheet-like member and the elastic member by cutting the sheet-like member continuous body and the elastic-member continuous body at the cutting target position after the fixing step and the joining-portion forming step,
the cutting step including attaching the elastic member to the sheet-like member by being sandwiched and pressed in the CD direction between the joining portions on the two sides,
the elastic member contracting in the direction of transport and attempting to expand in the CD direction due to being cut at the cutting target position.

2. The method for manufacturing a sheet-like member for an absorbent article according to claim 1, wherein
the method further comprises a welding step of forming a welded portion on two sides of the cutting target position in the direction of transport,
the welded portion being a portion that joins the pair of facing surfaces to each other,
the adhesive is applied in a predetermined application region of at least either of the elastic-member continuous body and one of the pair of facing surfaces, and
concerning a downstream welded portion being one of the welded portions on the two sides and being located on a downstream side in the direction of transport,
a downstream end of the application region in the direction of transport is located downstream in the direction of transport with respect to a downstream end of the downstream welded portion in the direction of transport.

3. The method for manufacturing a sheet-like member for an absorbent article according to claim 2, wherein
an upstream end of the application region in the direction of transport is located downstream in the direction of transport with respect to the downstream end of the downstream welded portion.

4. The method for manufacturing a sheet-like member for an absorbent article according to claim 2, wherein
an upstream end of the application region in the direction of transport is located in the direction of transport between the downstream end of the downstream welded portion and an upstream end of the downstream welded portion in the direction of transport.

5. The method for manufacturing a sheet-like member for an absorbent article according to claim 2, wherein
an upstream end of the application region in the direction of transport is located in the direction of transport between the cutting target position and an upstream end of the downstream welded portion in the direction of transport.

6. The method for manufacturing a sheet-like member for an absorbent article according to claim 2, wherein
an upstream end of the application region in the direction of transport is located upstream in the direction of transport with respect to the cutting target position.

7. The method for manufacturing a sheet-like member for an absorbent article according to claim 1, wherein
the method further comprises a welding step of forming a welded portion on two sides of the cutting target position in the direction of transport,
the welded portion being a portion that joins the pair of facing surfaces to each other,
the adhesive is applied in a predetermined application region of at least either of the elastic-member continuous body and one of the pair of facing surfaces, and
concerning a downstream welded portion being out of the welded portions on the two sides and being located on a downstream side in the direction of transport,
a downstream end of the application region in the direction of transport is located on upstream in the direction of transport with respect to a downstream end of the downstream welded portion in the direction of transport.

8. The method for manufacturing a sheet-like member for an absorbent article according to claim 7, wherein
an upstream end of the application region in the direction of transport is located downstream in the direction of transport with respect to an upstream end of the downstream welded portion in the direction of transport.

9. The method for manufacturing a sheet-like member for an absorbent article according to claim 7, wherein
an upstream end of the application region in the direction of transport is located in the direction of transport between the cutting target position and an upstream end of the downstream welded portion in the direction of transport.

10. The method for manufacturing a sheet-like member for an absorbent article according to claim 7, wherein
an upstream end of the application region in the direction of transport is located upstream in the direction of transport with respect to the cutting target position.

11. The method for manufacturing a sheet-like member for an absorbent article according to any of claims 4 to 10, wherein
the welded portions are provided so as to be overlapped with the application region when viewing the sheet-like member continuous body in a thickness direction that is orthogonal to both the direction of transport and the CD direction.

12. The method for manufacturing a sheet-like member for an absorbent article according to any of claims 2 to 11, wherein
at least one joining portion out of the joining portions is provided so as to be overlapped with the application region when viewing the sheet-like member continuous body in a thickness direction that is orthogonal to both the direction of transport and the CD direction.

13. The method for manufacturing a sheet-like member for an absorbent article according to any of claims 1 to 12, wherein
a plurality of the elastic-member continuous bodies are arranged side-by-side spacing between elastic-member continuous bodies that are adjacent in the CD direction,
in the fixing step, for each of the plurality of elastic-member continuous bodies,
the portion of the elastic-member continuous body that is to be the end portion of the elastic member in the direction of transport when cutting the elastic-member continuous body at the cutting target position is fixed to the at least one facing surface with use of the adhesive,
in the joining-portion forming step,
the joining portions respectively corresponds to the plurality of elastic-member continuous bodies, and
among the plurality of elastic-member continuous bodies, when one is defined as a first elastic-member continuous body, and another one is defined as a second elastic-member continuous body,
a tension (N) of the first elastic-member continuous body in the state of being stretched is higher than a tension (N) of the second elastic-member continuous body in the state of being stretched, and
a length in the direction of transport of the application region of the first elastic-member continuous body is larger than a length in the direction of transport of the application region of the second elastic-member continuous body.

14. A device for manufacturing a sheet-like member for an absorbent article,
the sheet-like member having an elastic member attached to the sheet-like member,
the sheet-like member being manufactured by inserting an elastic-member continuous body between a pair of mutually-opposing facing surfaces of a sheet-like member continuous body, and then cutting the sheet-like member continuous body at a cutting target position in a direction of transport of the sheet-like member continuous body,
the elastic-member continuous body being continuous in the direction of transport,
the sheet-like member continuous body being continuous in the direction of transport
the device comprising:
an adhesive application device that discharges an adhesive in order to fix the elastic-member continuous body to at least one facing surface out of the pair of facing surfaces with use of the adhesive,
the elastic-member continuous body being stretched in the direction of transport,
the adhesive application device discharging the adhesive so as to fix a portion of the elastic-member continuous body to the at least one facing surface with use of the adhesive,
the portion being a portion that is to be an end portion of the elastic member in the direction of transport when cutting the elastic-member continuous body at the cutting target position;
a joining-portion forming device that forms a plurality of joining portions spacing in the direction of transport, the joining portions joining the pair of facing surfaces to each other,
the joining-portion forming device forming the plurality of joining portions at positions on two sides of the elastic-member continuous body in a CD direction that intersects the direction of transport; and
a cutting device that produces the sheet-like member by cutting the sheet-like member continuous body at the cutting target position when the sheet-like member continuous body is located at a position downstream in the direction of transport with respect to the adhesive application device and the joining-portion forming device,
the elastic member being attached to the sheet-like member by being sandwiched and pressed in the CD direction between the joining portions on the two sides,
when the attaching, the elastic member being in a state of contracting in the direction of transport and attempting to expand in the CD direction due to being cut at the cutting target position by the cutting device.
